(19) Europäisches Patentamt
European Patent Office
Office européen des brevets

(11) EP 2 700 710 A1

(12) EUROPEAN PATENT APPLICATION
published in accordance with Art. 153(4) EPC

(43) Date of publication:
26.02.2014 Bulletin 2014/09

(21) Application number: 12773570.2

(22) Date of filing: 27.03.2012

(51) Int Cl.:
*C12N 5/12* (2006.01) *C07K 16/14* (2006.01)
*C07K 19/00* (2006.01) *C12N 15/13* (2006.01)
*C12N 15/62* (2006.01) *C12N 15/63* (2006.01)
*C12N 15/70* (2006.01) *C02F 1/50* (2006.01)
*C12R 1/89* (2006.01)

(86) International application number:
PCT/CN2012/073120

(87) International publication number:
WO 2012/142899 (26.10.2012 Gazette 2012/43)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 21.04.2011 CN 201110100775
10.06.2011 CN 201110155221

(71) Applicant: Protein Design Lab, Ltd.
Beijing 100095 (CN)

(72) Inventor: QIU, Xiaoqing
Beijing 100095 (CN)

(74) Representative: Herrmann, Uwe et al
Lorenz - Seidler - Gossel
Widenmayerstrasse 23
80538 München (DE)

# (54) ANTI-CYANOBACTERIA RECOMBINANT ANTIBODY POLYPEPTIDE, GENE THEREOF, AND PREPARATION METHOD THEREOF

(57) Provided are a hybridoma cell CGMCC No.4783 that secretes a monoclonal antibody of an anti-cyanobacteria cell surface antigen, and the secreted monoclonal antibody thereof. Also provided are an anti-cyanobacteria recombinant antibody poly - peptide, encoding gene, preparation method and use thereof. The anti-cyanobacteria recombinant antibody polypeptide is composed of an anti-cyanobacteria antibody mimetic polypeptide operably linearly connecting to the carboxyl terminal of an Escherichia coli polypeptide. The anti-cyanobacteria antibody mimetic polypeptide is a polypeptide with cyanobacteria identifying and binding cap - ability designed based on an antigen binding fragment of the monoclonal antibody secreted by the CGMCC No.4783 hybridoma cell. The anti-cyanobacteria recombinant antibody polypeptide directly form an ion channel on the cell membrane of a cyanobacteria to kill the cyanobacteria, targeted killing the cyanobacteria (prokaryote) without killing other beneficial eukaryotic cell algae.

EP 2 700 710 A1

## Description

### TECHNICAL FIELD

The invention relates to biotechnology in environment protection, especially a anti-cyanobacteria recombinant antibody polypeptide, gene thereof, and preparation method thereof.

### BACKGROUND

Cyanobacteria proliferation and water pollution caused by water eutrophication are the biggest harm for the worldwide water environment that lead to great economic loss and irreparable damage towards the biosphere of the earth. Along with the accelerated industrialization and urbanization by the economic development of China, environment pollution and degeneration are severer and severer, and bionomic control on water environment has become a crutial problem to be faced and solved.

The present antibacterial agents are hardly effective against cyanobacteria, a prokaryocyte that belongs to Phylum X of cyanobacteria, Domain Bacteria.. Currently, there is only heavy metal chemical reagent, for example, copper sulfate and etc, which can control cyanobacteria. However, in practical use against cyanobacteria, the overdosed and repeated use of drugs due to their limited effects destroys against other beneficial algae, aquatic plants, organisms, and brings irreversible and perpetual wreck to the environment, as well as heavy metal agent residue with increasing amount in both the environment and agricultural products. Therefore, it is of great demand for the development of high-effective and safe anti-cyanobacterial drugs.

### SUMMARY

In one aspect, the present invention provides genes, recombinant plasmids, and polypeptides of a novel anti-cyanobacteria recombinant antibody polypeptide; said polypeptides are capable of specifically killing cyanobacteria cells but not damaging other algae cells, plants and animals. Another aspect of the present invention is to provide methods for preparing said anti-cyanobacteria recombinant antibody polypeptides.

Provided is a hybridoma with a depository number of CGMCC No.4783.

Also provided is a monoclonal antibody secreted by said hybridoma.

Also provided is an antibody mimics polypeptide with function of recognizing and binding cyanobacteria, which is an antigen-binding fragment of said monoclonal antibody, or a small molecular polypeptide that is designed on the basis of functional regions of said antigen-binding fragment of said monoclonal antibody.

Said antibody mimic polypeptide, its amino acid sequence sets forth in SEQ ID NO. 3.

Also provided is gene encoding any one of said antibody mimic polypeptides?

Said gene has a nucleotide sequence set forth in SEQ ID NO.2.

Also provided is an anti-cyanobacteria recombinant antibody polypeptide, constructed by operably and linearly connecting said antibody mimic polypeptide capable of recognizing and binding cyanobacteria to the C-terminus of colicin polypeptide, and said colicin is selected from colicin E1, Ia, Ib, A, B or N.

Said anti-cyanobacteria recombinant antibody polypeptide has an amino acid sequence set forth in SEQ ID NO.7.

Also provided is a gene encoding said any one of anti-cyanobacteria recombinant antibody polypeptide.

Said gene has a nucleotide sequence shown in SEQ ID NO.6.

Also provided is a recombinant expression vector, which comprises the gene encoding said anti-cyanobacteria recombinant antibody polypeptide.

Also provided is a method for preparing said anti-cyanobacteria recombinant polypeptide, with steps as follows: transducting the gene encoding the anti-cyanobacteria recombinant antibody polypeptide into an *E. coli* expression system, cultivating the transducted *E. coli,* and separating the polypeptide expressed by *E. coli.*

Also provided is the use of said anti-cyanobacteria recombinant antibody polypeptide on the control of water eutrophication.

The invention provides an anti-cyanobacteria recombinant antibody polypeptide, which is constructed from colicin polypeptide and an antibody mimic polypeptide against cyanobacteria cell surface antigen. In said polypeptide molecule, the antibody mimetics against cyanobacteria cell surface antigen recognizes and binds to cyanobacteria, leading colicin of the molecule to attack the recognized cyanobacteria cell. With an antibody mimetic recognizing cyanobacteria cell surface antigen specifically, the polypeptide of the invention attacks cyanobacteria cells with no damage to other water organisms and no water pollution, thus being environment-protecting and safe on control of water cyanobacteria pollution. The antibody mimetic in the anti-cyanobacteria recombinant antibody polypeptide of the invention is an antibody mimetic capable of recognizing and binding to cyanobacteria, and is designed based on the amino acid sequences and nucleotide sequences of the light and heavy chain of the antigen-binding fragment (Fab) of the monoclonal antibody secreted by hybridoma CGMCC No.4783, such as mimic antibodies well known in the art, *e.g.,* single-chain antibody and small molecular antibody mimetics, *etc.* In the invention, the designed antibody mimetic just need to be capable of recognizing the antigen, as the only task of the antibody mimetics is to guide the recombinant polypeptide molecule to target cell surface, and build an ion tunnel on the cell membrane by colicin in the recombinant polypeptide, thereby resulting in the target cell death due to intracellular substance leak. Therefore, any recombinant polypeptides constructed by linking a colicin to an antibody mimetic that is designed on the basis of said monoclonal antibody and can recongnize the target antigen, will fall into the claimed scope of the invention.

In an embodiment of the invention, the antibody mimetic is a 28-peptide antibody mimetics constructed by linking domains of $V_H$CDR1, $V_H$FR2 and $V_L$CDR3 of a monoclonal antibody against surface antigen of cyanobacteria protoplast (secretd by hybridoma CGMCC No.4783) in order of $V_H$CDR1-$V_H$FR2 -$V_L$CDR3. Indoor and outdoor experiments proved that, the anti-cyanobacteria polypeptide constructed by said antibody mimetics has biological activity of moveing around and automatically looking for the cyanobacteria cell surface antigen in water. The 28-peptide antibody mimetics has advantages that it is easily to be cloned, expressed, and has recognizing activity of the original antibody; it can kill cyanobacteria by guiding the colicin of the recombinant polypeptide to target cell membrane to build an ion tunnel. Said recombinant polypeptide has more ecological and environmental safety, compared with current drugs on controlling water pollution (e.g., copper sulfate).

In the invention, the colicin for the construction of the anti-cyanobacteria recombinant antibody polypeptide may be selected from colicin E1, Ia, Ib, A, B or N that could form ion channel. The recombinant polypeptide constructed by anti-cyanobacteri antibody mimetics and colicin Ia is verified by examples of the invention. However, with the property of forming an ion channel on the cell membrane of cyanobacteria, any of these colicins according to conception of the invention may be linked linearly to anti-cyanobacteria antibody mimetics for constructing the recombinant polypeptide to carry out the invention, that is, to provide recombinant polypeptides that kill cyanobacteria.

The invention also provides preparation method of the recombinant plasmid comprising the gene of recombinant polypeptide, wherein the nucleotide sequence encoding antibody mimetics is inserted into the C-terminus of colicin by double strand oligonucleotides site-directed mutagenesis, to form the recombinant plasmid of the invention. Taking colicin Ia as an example, the recombinant plasmid of the invention is constructed by inserting the nucleotide sequence encoding antibody mimetics into the site after amino acid 626 of colicin Ia gene (the C-terminus). The original vector pSELECT-1 is purchased from Promega Corp., which contains genes of colicin Ia and Immunity protein. In Example 1 of the present invention, several pairs of primer sequences are designed for genes of antibody mimetics. The recombinant plasmid, e.g. pCHCcyanoMA1, is prepared according to the instruction of the kit from Strategene Corp. by double strand oligonucleotides site directed mutagenesis.

In another aspect of the invention, provided is a preparation method of said anti-cyanobacteria polypeptide of the invention, wherein a recombinant plasmid comprising a gene encoding anti-cyanobacteria recombinant antibody polypeptide is obtained by operably linking a gene encoding antibody mimetics with a colicin gene, and the obtained recombinant plasmid is transfected into an *E. coli* engineering bacteria to obtain a engineering bacteria cell that may produce anti-cyanobacteria recombinant antibody polypeptide. Said anti-cyanobacteria polypeptide of the invention is then obtained from separation and purification with ion-exchange column.

Said anti-cyanobacteria polypeptide of the invention may be used in the control of water pollution. Drug combinations suitable for environment may be prepared by adding vectors, excipients, or other alternative components into the polypeptide of the invention, wherein the mechanism is as follows: antibody mimetics within the polypeptide that recognize cyanobacteria cell surface antigen guide colicin to the cyanobacteria cell membrane with the hydrophobic fragment of ion channel domain in colicin inserted into, to form an ion channel, thereby cyanobacteria cell death is incurred by intracellular substance leak.

Said anti-cyanobacteria polypeptide of the invention has an advantage of target specificity compared with traditional anti-cyanobacterial drugs, thus it does not attack other algae, plants and animal cells; toxicity and untoward effect far less than traditional anti-cyanobacterial drugs. Furthermore, since the ion channel is directly formed on cyanobacteria cell membrane to kill cyanobacteria cells, it is difficult for cyanobacteria cells to repair the geometrical damages on cell membrane by way of gene mutation and protein expression, in the short time of intracellular substance leak through anti-cyanobacteria polypeptide ion channel to cyanobacteria cell death. Therefore, it is with low probability that cyanobacteria cells obtains drug resistance of the anti-cyanobacteria polypeptide of the invention, which indicates there is a promising application prospect to said anti-cyanobacteria polypeptide of the invention.

Deposit information of hybridoma:

Deposit Number: CGMCC No.4783
Deposit Name: Monoclonal antibody hybridoma against cyanobacteria cell surface antigen
Depository Date: April 4, 2011
Depositary institution: China General Microbiological Culture Collection Center, CGMCC

Address: No.3, Courtyard No. 1, Beichen West Road, Chaoyang District, Beijing

DESCRIPTION OF DRAWINGS

FIG.1 schematic illustration of the structure of recombinant plasmid pCHCcyanoMA1 comprising genes of antibody mimetics and colicin Ia

FIG. 2 schematic illustrates the structure of the anti-cyanobacteria recombinant antibody of present invention.

FIG.3 schematic illustration of the structure of recombinant plasmid pCHCcyanoMA2 comprising genes of antibody mimetics and colicin Ia with reversed N-C order in part.

FIG.4 the experiment results about the inhibition of the anti-cyanobacteria polypeptide of the invention on *Microcystis aeruginosa* cultivated in liquid medium,

Wherein, **A,** blank control; **B,** 50 μg/ml of ampicillin; **C,** broad 50 μg/ml of spectrum pheromone (Ph-NM); **D,** 50 μg/ml of anti-cyanobacteria polypeptide (1); **E,** 50 μg/ml of anti-cyanobacteria polypeptide (2).

FIG.5 the experiment results about the inhibition of the anti-cyanobacteria polypeptide (1) of the invention on *Microcystis aeruginosa* cultivated in liquid medium

Wherein, **A,** result of treating for 48 hours; **B,** result of treating for 72 hours; **C,** result of treating for 96 hours.

Wherein, there are 6 flasks in each column, from left to right, namely as follows: **1,** control; **2,** 0.1 μg/ml of anti-cyanobacteria polypeptide (1); **3,** 0.5 μg/ml of anti-cyanobacteria polypeptide (1); **4,** 1 μg/ml of anti-cyanobacteria polypeptide (1); **5,** 5 μg/ml of anti-cyanobacteria polypeptide (1); **6,** 10 μg/ml of anti-cyanobacteria polypeptide (1).

FIG.6 the experiment results about the inhibition of the anti-cyanobacteria polypeptide (1) of the invention on *Scenedesmus* cultivated in liquid medium.

Wherein, there are 6 flasks in each column which are treated for 10 days, from left to right, namely as follows: **1,** control; **2,** 1 μg/ml of anti-cyanobacteria polypeptide (1); **3,** 5 μg/ml of anti-cyanobacteria polypeptide (1); **4,** 10 μg/ml of anti-cyanobacteria polypeptide (1); **5,** 15 μg/ml of anti-cyanobacteria polypeptide (1); **6,** 20 μg/ml of anti-cyanobacteria polypeptide (1).

FIG. 7 the experiment results about the inhibition of the anti-cyanobacteria polypeptide (1) of the invention on *Microcystis aeruginosa* and *Scenedesmus* cultivated in liquid medium. Wherein, the result of *Microcystis aeruginosa* is shown in the upper part, and that of *Scenedesmus* is shown in the lower part.

FIG. 8 the experiment results about the inhibition of the anti-cyanobacteria polypeptide of the invention on *Microcystis aeruginosa, Anabaena, Chlorella,* and *Scenedesmus* cultivated in liquid medium.

Wherein, the left flask is control, and the right one is 35 μg/ml of anti-cyanobacteria polypeptide (1). Wherein A: *Microcystis aeruginosa;* B: *Anabaena;* C: *Chlorella;* D: *Scenedesmus.*

FIG.9 the experiment result about the inhibition of the anti-cyanobacteria polypeptide of the invention on the natural water polluted by cyanobacteria.

Wherein, **A,** blank control; **B,** 1 μg/ml of anti-cyanobacteria polypeptide (1); **C,** 5 μg/ml of anti-cyanobacteria polypeptide (1); **D,** 10 μg/ml of anti-cyanobacteria polypeptide (1); **E,** 0.7 μg/ml of copper sulfate.

FIG.10 some respects of results of the inhibition experiment in Fig. 9

Wherein, **A,** changes of optical density in water; **B,** changes of Chlorophyll a in water; **C,** pH changes in water; **D,** changes of dominant algae population in water.

Wherein, **D-1,** Control group; **D-2,** the group treated by 1 μg/ml of anti-cyanobacteria polypeptide (1); **D-3,** the group treated by 0.7 μg/ml of copper sulfate.

FIG.11 microscopic observation (magnified by 400-fold) of the inhibition of the anti-cyanobacteria polypeptide on natural water polluted by cyanobacteria in Fig. 9

Wherein, changes of algae (*Anabaena*) that is treated by control, 1 μg/ml of anti-cyanobacteria polypeptide (1) and 0.7 μg/ml of copper sulfate in water on the first Day 1 **(A),** the second Day **(B), the third** Day **(C),** the fourth Day **(D)**, the fifth Day **(E)** are shown respectively.

## DETAILED DESCRIPTION

### Example 1 Construction of plasmid expressing anti-cyanobacteria polypeptide and preparation of anti-cyanobacteria recombinant polypeptide

Material: Hybridoma CGMCC No.4783.

Step 1. Obtaining of the nucleotide sequence and amino acid sequence of antibody mimetics: the monoclonal antibody secreted by hybridoma was collected and sequenced by using conventional methods to obtain the sequence of heavy chain and light chain of antigen-binding fragment Fab; the amino acid sequence of antibody mimetics was designed on the basis of the amino acid sequence of complementarity-determining regions, as shown in SEQ ID NO. 3. The nucleotide sequence encoding said antibody mimetics is set forth in SEQ ID NO.2.

Step 2. The original vector is the plasmid pSELECT-1 (purchased from Promega Corp.), which carries genes of colicin Ia and Immunity protein (said genes are loaded in the lab where the experiments of this invention are conducted). The gene encoding anti-cyanobacteria antibody mimetics (nucleotide sequence shown in SEQ ID NO.2 in the sequences listing) was inserted after the site of I626 on the gene of colicin Ia by double-strand oligonucleotide Site-Directed Mutagenesis technology (QuickChange™ Kit, Strategene Corp.), to obtain a recombinant plasmid pCHCcyanoMA1 (as shown in Fig. 1). The recombination plasmid was then transfected into the engineering bacteria of *E. coli* B834 (DE3) to prepare the polypeptide. Said anti-cyanobacteria polypeptide with amoni acid sequence shown in SEQ ID NO.7 (hereinafter referred to as "anti-cyanobacteria polypeptide (1)") and molecular weight about 70,000 was obtained.

The process of double-strand oligonucleotide site-directed mutagenesis was performed according to the manual of Strategene QuickChange Site-Directed Mutagenesis Kit (catalog#200518).

1. Preparation of reactants for site-directed mutagenesis:

    5 μl 10 x buffer
    2 μl (10 ng) wild-type colicin Ia plasmid
    1 μl (125ng) 5'-3' oligonucleotide primers as designed
    1 μl (125ng) 5'-3' oligonucleotide primers as designed
    1 μl dNTP
    Double-distilled water 50 μl
    1 μl pfu
    (All is provided in the Kit except for the plasmid, primers and double-distilled water)

2. PCR amplification, amplification conditions: 20 cycles of denaturation at 95°C for 35 seconds, anneal at 53°C for 70 seconds, and extension at 68°C for 17 minutes;
3. 1 μl of endonuclease Dpn 1 was added to digest DNA chain (37°C, 1 hour); 1 μl of reactant and 50 μl of HMS174 competent cell were incubated together in ice for 30 minute, and then incubated in ice for 2 minute after a heat shock at 42°C for 45 seconds;
4. 0.5 ml of culture medium NZY was added, and cultivated by shaking at 37°C, 220 rpm for 1 hour. 50-100 μl of reactant was spread on LB medium plate plus 1% agar and 50 μg/ml of ampicillin for cultivating overnight at 37°C;
5. Colonies were picked up after cultivating for 18 hours. Plasmid was extracted by using extraction Kit of Qiagene Corp. or Gibco Corp., and then sequenced to confirm if the mutation is successful;
6. 50 ng of recombination plasmid was incubated with 50 μl of the prepared E. coli B834 (DE3) competent cells in ice for 30 minute, and heat shocked at 42°C for 30 second. 50-100 μl of the reactant was added with 0.5 ml of SOC culture medium, cultivated by shaking at 37°C, 220 rpm for 1 hour and spread on LB medium plate plus 1% agar and 50 μg/ml of ampicillin for incubating at 37°C for 12-16 hours. Afterwards, single colony was picked up;
7. Bacteria were amplified in 8-16 liters of LB medium at 250 rpm, 37°C for 6-8 hours. The bacterium was precipitated by centrifugation at 6000 g, 4°C for 20 minutes, resuspended with 50-80 ml of 50 mM Borate, 2mM EDTA (pH 9.0) at 4°C; 250 microliters of 0.2M PMSF was added and treated with ultrasonication at 4°C, 400W for 2 minutes. Bacterium debris was high-speed centrifugated at 4°C, 75,000 g for 1.5 hours. The supernatant was added with streptomycin sulphate to precipitate DNA, dialyzed overnight in dialysis bag with molecular weight of 15,000 as well as with 10 L of 50 mM Borate, 2mM EDTA, pH 9.0 buffer at 4°C, and then loaded on a CM column (Amersham Biosciences). The column was eluted by a buffer of 50 mM Borate, 0.3 M NaCl, 2mM EDTA, pH 9.0 to obtain the anti-cyanobacteria recombinant polypeptide (1) (See Fig. 2)

Sequences of double strand oligonucleotides designed for the preparation on genes of antibody mimetics of the plasmid pCHCcyanoMA1 are as follows:

pCHCcyanoMA1
1.5'-3'

gcg aat aag ttc tgg ggt att TCT TAT TGG ATG CAG TGG GTT AAG CAA taa ata aaa tat aag aca ggc

3'-5'

gcc tgt ctt ata ttt tat tta TTG CTT AAC CCA CTG CAT CCA ATA AGA aat acc cca gaa ctt att cgc

2.5"-3'

tat tgg atg cag tgg gtt aag caa AGA CCG GGG CAA GGG CTG GAG TGG ATT GGC taa ata aaa tat aag aca ggc

3'-5'

gcc tgt ctt ata ttt tat tta GCC AAT CCA CTC CAG CCC TTG CCC CGG TCT ttg ctt aac cca ctg cat cca ata

3.5'-3'

ggg caa ggg ctg gag tgg att ggc CAG CAG TAT YGG TCT ACG CCC CCG TGG ACG taa ata aaa tat aag aca ggc

3'-5'

gcc tgt ctt ata ttt tat tta CGT CCA CGG GGG CGT AGA CCA ATA CTG CTG gcc aat cca ctc cag ccc ttg ccc

**Example 2 Construction of a control plasmid expressing anti-cyanobacteria polypeptide and preparation of the control anti-cyanobacteria polypeptide**

The original vector is the plasmid pSELECT-1 (purchased from Promega Corp.), which carries genes of colicin Ia, and Immunity protein (said genes are loaded in the lab where the experiments of this invention are conducted). The gene encoding anti-cyanobacteria antibody mimetics (nucleotide sequence shown in SEQ ID NO.4 in the sequence list) was inserted after the site of 1626 on the gene of colicin Ia by double-strand oligonucleotide Site-Directed Mutagenesis technology (QuickChange™ Kit, Strategene Corp.) to obtain recombinant plasmid pCHCcyanoMA2 (as shown in Fig. 3). The recombination plasmid was transfected into the engineering bacteria of *E. coli* B834 (DE3) to prepare the polypeptide. Said anti-cyanobacteria polypeptide whose sequence list is shown in SEQ ID NO.9 (hereinafter referred to as "anti-cyanobacteria polypeptide (2)") and the molecular weight is about 70,000 was obtained.

Sequences of double strand oligonucleotides for the preparation on genes of antibody mimetics of the plasmid pCHCcyanoMA2 was designed and prepared according to the method in Example 1.

**Example 3 Experiment results of inhibition of anti-cyanobacteria polypeptide on *Microcystis aeruginosa***

Experiment (1)

Step 1. Preparation of algae for experiment: *Microcystis aeruginosa* FACHB-978 is purchased from the Institute of Hydrobiology, Chinese Academy of Sciences.

Cultivation method of the algae: algae seed was inoculated with an initial cell density of $5 \times 10^4$/mL into a 500 ml conical flask containing 250 ml of BG11 medium and placed into an illumination incubator, wherein the conical flask was covered with breathable film to prevent pollutions. Algae were cultivated at 25 °C with illumination intensity of (2500±10%) lux and light/dark ratio of 12h/12h. Conical flasks were shaked and position-exchanged once every 2-3 hours. Samples were taken and tested once every 24 hours since the 5th day after the inoculation. The next step could be performed till the cell density reached about $10^6$ /mL.

Step 2. 1 ml of prepared cyanobacteria liquid is added respectively into 5 tubes, namely, **A, B, C, D,** and **E,** and then inhibitors were added as follows: **A,** blank control; **B,** 50μg/ml of ampicillin; **C,** 50μg/ml of broad spectrum pheromone (Ph-AM1 disclosed in Patent Application No. CN200910092128.4); **D,** 50μg/ml of anti-cyanobacteria polypeptide (1); **E,** 50μg/ml of anti-cyanobacteria polypeptide(2). After shaking cultivation at low speed for 12hours in a incubator, the 5 tubes were respectively added with acridine orange (50nM) and propidium iodide (600nM) for incubating for 30 min; several microliters were sampled onto a slide to observe the alive stained cyanobacteria by a fluorescence microscope (Nikon 90i with filter of DM505 and DM565).

Results are shown in Fig. 4. It's manifested by results that only anti-cyanobacteria polypeptide (1) killed the cultivated *Microcystis aeruginosa,* whereas none of anti-cyanobacteria polypeptide (2), ampicillin and Ph-NM showed effects on the cultivated *Microcystis aeruginosa.*

Experiment (2)

Step 1. It was same as in Step 1 in Experiment (1).

Step 2. Anti-cyanobacteria polypeptides were added by concentration gradient into 6 of 500 ml conical flasks filled with algae. The anti-cyanobacteria polypeptide was added to *Microcystis aeruginosa* in doses of 0, 0.1, 0.5, 1, 5, and 10 μg/ml. The inhibition on cyanobacteria was observed every day.

Results were shown in Fig. 5. Because the concentration of anti-cyanobacteria polypeptide (1) was different in each group, the time of colour changing from green to light yellow of *Microcystis aeruginosa* liquid in each conical flask was proportional to the concentration of anti-cyanobacteria polypeptide (1). However, it was observed visually that *Microcystis aeruginosa* in all treatment could be killed effectively in 72 hours.

The above result showed that anti-cyanobacteria polypeptide (1) is capable of target killing the cultivated *Microcystis aeruginosa,* whereas anti-cyanobacteria polypeptide (2) hardly shows any effect.

Experiment (3)

Material: *Scenedesmus obliqnus* FACHB-417 is purchased from the Institute of Hydrobiology, Chinese Academy of Sciences, and cultivated according to the method in Step 1 of Experiment (1).

Step 2. Anti-cyanobacteria polypeptide (1) was added into 6 of 500 ml conical flasks with algae by concentration gradient 0, 1, 5, 10, 15, and 20 μg/ml respectively. The inhibition on *Scenedesmus* was observed every day.

Results are shown in Fig. 6. Although the dose of anti-cyanobacteria polypeptide (1) was 10 times higher than that added in *Microcystis aeruginosa,* no killing effect of anti-cyanobacteria polypeptide (1) on *Scenedesmus* was observed visually during the experiment (190 hours).

Experiment (4)

0.1 mL of culture liquid is taken from each flask of the Experiment (1) - (3) and placed in a blood counting chamber for observing and counting under a microscope. It's shown by the algae cell number in Fig. 7 that, compared to the control, 1 μg/ml of anti-cyanobacteria polypeptide (1) can kill *Microcystis aeruginosa* in 72 hours, whereas 1 μg/ml of anti-cyanobacteria polypeptide (1) hardly showed any effect against *Scenedesmus.*

**Example 4. Experiment of anti-cyanobacteria polypeptide against *Microcystis aeruginosa, Anabaena, Chlorella* and *Scenedesmus* in liquid culture medium.**

*Microcystis aeruginosa, Anabaena, Chlorella* and *Scenedesmus* are purchased from the Institute of Hydrobiology, Chinese Academy of Sciences, cultivated and treated according to the methods in Step 1, Experiment (1) of Example 3.

40 ml of the algae liquid with cell density of $10^6$/mL was distributed into two 300ml conical flasks, and added respectively with **A:** blank control liquid with same amount, **B:** 35 μg/ml of anti-cyanobacteria polypeptide (1), and cultivated for 4 days. The growth was observed.

Results were shown in Fig. 8. After 24 hours, the color of algae liquid of *Microcystis aeruginosa* and *Anabaena* turned light and became clear on the fourth Day, whereas the colour and turbidity of *Chlorella* and *Scenedesmus* liquid stayed constant. It's illustrated by the results that, anti-cyanobacteria polypeptide 1 inhibited the growth of the *Microcystis aeruginosa* and *Anabaena,* but did not inhibit the growth of *Chlorella* and *Scenedesmus.*

The above results proved that anti-cyanobacteria polypeptide (1) is capable of target killing cyanobacteria.

**Example 5 Experiment of killing cyanobacteria in natural water by anti-cyanobacteria polypeptide**

Natural water seriously polluted by cyanobacteria was taken from a shrimp breeding pool of Suzhou Yangcheng Lake, 25~35 L of the water was distributed into plastic breeding basins of 60 x 40 x 30 cm, with 10 min of air supply by bumping in every 20 min, temperature automatically adjusted to 27 °C, illumination intensity of (2500±10%) lux and light/dark ratio of 12h/12h. Water was sampled every day to detect the pH, absorbance at 650 nm, and Chlorophyll a absorbance (See Fig. 9).

The absorbance at 650 nm was detected by placing the sample liquid into a visible spectrophotometer (See Fig. 10).

Chlorophyll a content was detected by using hot ethanol- freeze thawing-spectrophotometry method (See Fig. 10). The absorbances at 650 and 700 nm were detected by a spectrophotometer to calculate concentration of Chlorophyll a, with a formula of:

$$[\mathrm{Chla}]=[12.12\,(D664-D750)-1.58(D647-D750)-0.08(D630-D750)]\,V\mathrm{E}/V\mathrm{S}\cdot \mathrm{d}.$$

PH, absorbance at 650 nm, and Chlorophyll a absorbance in water of the control group remained high during the experiment, whereas those of the anti-cyanobacteria polypeptide (1) group and copper sulfate group decreased dramatically since the second day.

30 ml of water was sampled every day, fixed with formalin, and then concentrated for 10 times by centrifugation. 0.1 mL was taken into a blood counting chamber to be observed and counted under a microscope (See Fig. 11). It's displayed by microscopic examination that, on the first day, plenty of blossoming *Anabaena* could be seen in control group, anti-cyanobacteria polypeptide (1) group and copper sulfate group; whereas since the second day, *Anabaena* in anti-cyanobacteria polypeptide (1) group and copper sulfate group decreased and fragmented greatly in water; and no *Anabaena* could be seen in anti-cyanobacteria polypeptide (1) group and copper sulfate group on the fifth day, but plenty *of Anabaena* in control group were still alive.

Changes of algae shape was observed under a microscope, and various algae were counted to detect the dynamic changes about dominant population of floating plants in water (See Fig. 10). In control group, *Anabaena* and *Microcystis aeruginosa* were dominant algae all the time; in anti-cyanobacteria polypeptide (1) group, *Anabaena* and *Microcystis aeruginosa* decreased gradually while *Chlorella* became dominant; in copper sulfate group, *Anabaena* and *Microcystis aeruginosa* decreased gradually, and another *Cyanobacteria,* the *Oscillatoria,* became dominant.

**Table 1 Number count and shape change of floating plants treated by anti-cyanobacteria polypeptide (/L)**

| | 1st Day of treating | 2nd Day | 3rd Day | 4th Day | 5th Day |
|---|---|---|---|---|---|
| Control group | 32745 | 26363<br>No change in algae shape | 33194<br>No change in algae shape | 41068<br>No change in algae shape | 17954<br>No change in algae shape |
| 1 mg/L of polypeptide | 50394 | 43981<br>Most *Anabaena* became single cells or short chains of 2-5 cells | 10624<br>Most of *Anabaena* become short chains of 1-5 cells, without long chain | 5370<br>Most of *Anabaena* become short chains of 1-5 cells with little amount | 3432<br>Almost no *Anabaena* cells |
| 5 mg/L of polypeptide | 43987 | 40828<br>Same as the change treated by 1 mg/L polypeptide | 20744<br>Same as the change treated by 1 mg/L polypeptide | 9890<br>Same as the change treated by 1 mg/L polypeptide | 3680<br>Almost no *Anabaena* cells |
| 10 mg/L of polypeptide | 48101 | 41787<br>Same as the change treated by 1 mg/L polypeptide | 23443<br>Same as the change treated by 1 mg/L polypeptide | 14214<br>Same as the change treated by 1 mg/L polypeptide | 4902<br>Almost no *Anabaena* cells |

(continued)

| | 1st Day of treating | 2nd Day | 3rd Day | 4th Day | 5th Day |
|---|---|---|---|---|---|
| 20 mg/L of polypeptide | 45588 | 39194 Same as the change treated by 1 mg/L polypeptide | 33453 Same as the change treated by 1 mg/L polypeptide | 4800 Almost no *Anabaena* cells | 3508 Almost no *Anabaena* cells |
| 0.7 mg/L of copper sulfate | 22930 | 1266 all *Anabaena* became single cells | 499 Almost all the algae became organic detritus | 305 Few algae | 379 Few algae |

Results are shown in Figs. 9-11 and Table 1. It's manifested by results that 0.7 ppm copper sulfate killed all kinds of algae without selection. However, anti-cyanobacteria polypeptide (1) has targeting inhibition on the growth of cyanobacteria *(Anabaena, Microcystis aeruginosa*) in water, but has little influence on the growth of other phytoplankton.

## Claims

1. A hybridoma with a depository number of CGMCC No.4783.

2. A monoclonal antibody secreted by said hybridoma of claim 1.

3. An antibody mimetic polypeptide with function of recognizing and binding to cyanobacteria, said antibody mimetic polypeptide being an antigen-binding fragment of said monoclonal antibody of claim 2, or a small molecular polypeptide that is designed on the basis of functional regions of said antigen-binding fragment and artificially expressed and isolated.

4. The antibody mimetic polypeptide according to claim 3, its amino acid sequence sets forth in SEQ ID NO. 3.

5. A gene encoding the antibody mimetic polypeptide of claim 3 or 4.

6. The gene according to claim 5, its nucleotide sequence sets forth in SEQ ID NO.2.

7. A anti-cyanobacteria recombinant antibody polypeptide, constructed by operably and linearly connecting said antibody mimetic polypeptide with function of recognizing and binding to cyanobacteria of claim 3 or 4 to the C-terminus of colicin polypeptide, said colicin is selected from colicin E1, Ia, Ib, A, B or N.

8. The anti-cyanobacteria recombinant antibody polypeptide according to claim 7, its amino acid sequence sets forth in SEQ ID NO.7.

9. A gene encoding the anti-cyanobacteria recombinant antibody polypeptide.

10. The gene according to claim 9, its nucleotide sequence sets forth in SEQ ID NO.6.

11. A recombinant expression vector comprising the gene encoding the anti-cyanobacteria recombinant antibody polypeptide.

12. A method of preparing the anti-cyanobacteria recombinant polypeptide of claim 7 or 8, comprising steps of: transforming the gene encoding the anti-cyanobacteria recombinant antibody polypeptide into an *E. coli* expression system, cultivating the transformed *E. coli,* and separating the polypeptide expressed by *E. coli.*

13. Application of said anti-cyanobacteria recombinant antibody polypeptide of claim 7 or 8 on control water eutrophication.

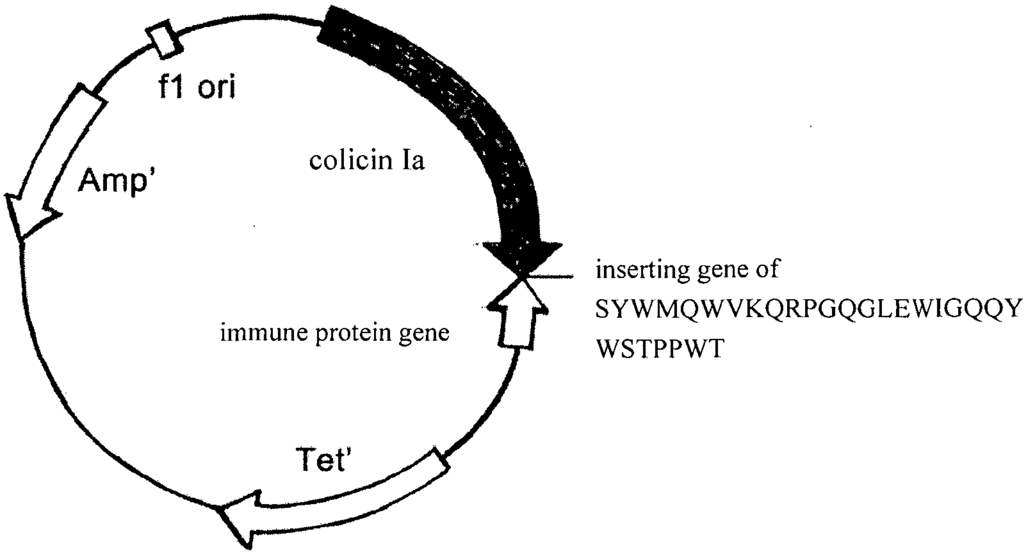


Fig. 1

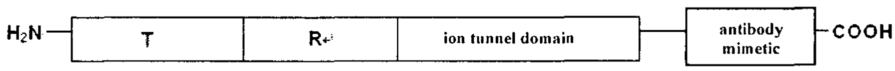


Fig. 2

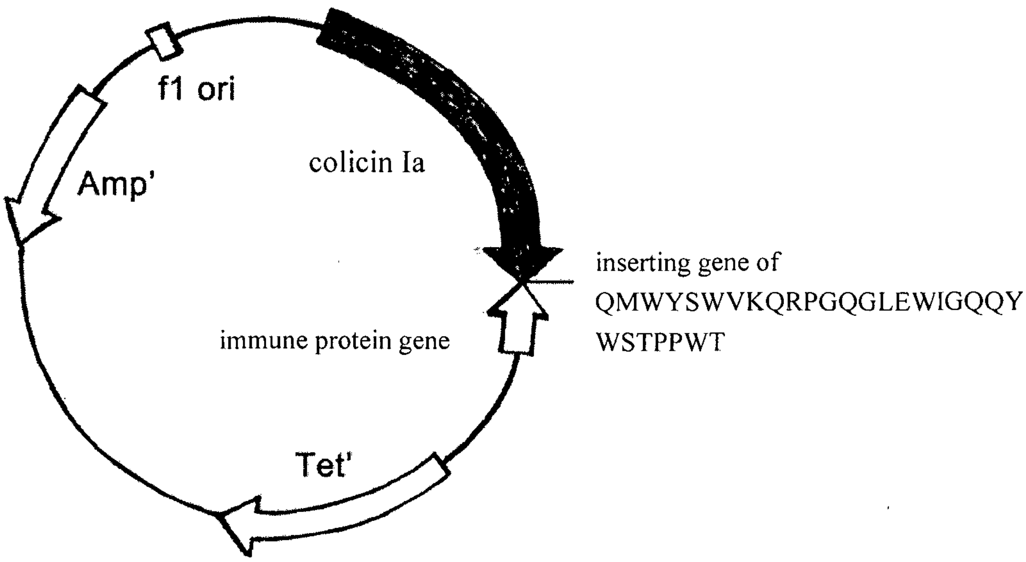


Fig. 3

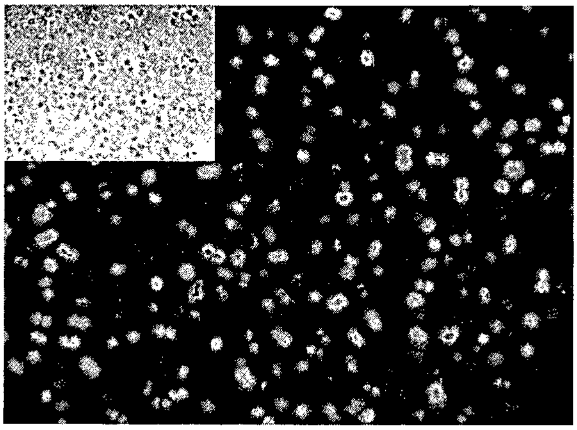

Fig. 4A

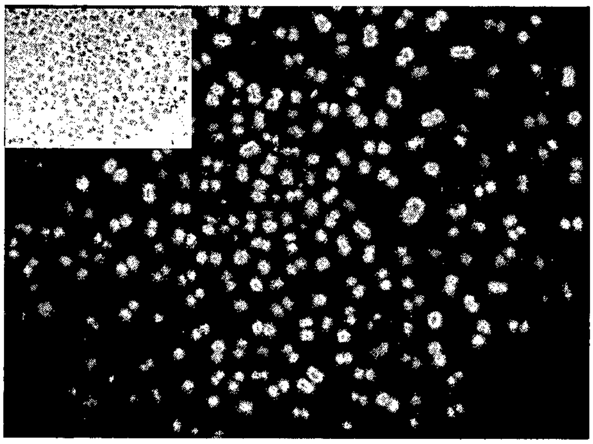

Fig. 4B

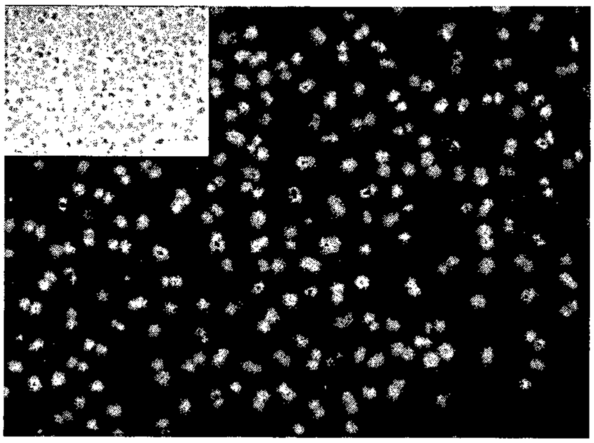

Fig. 4C

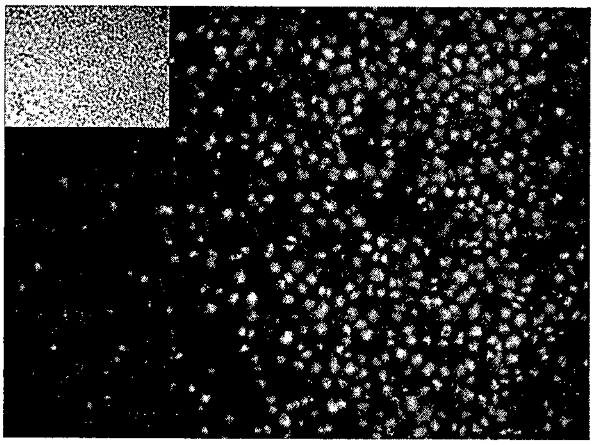

Fig. 4D

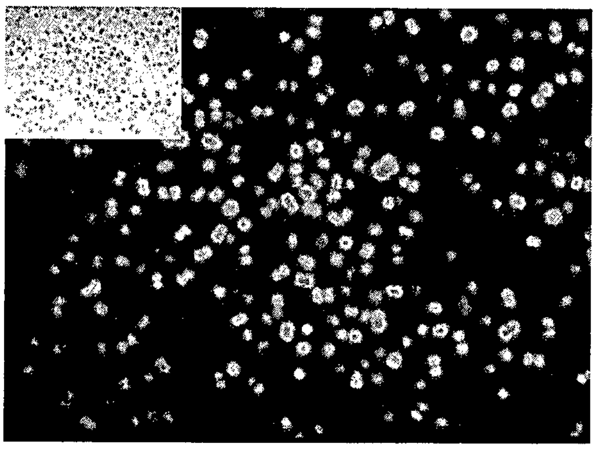

Fig. 4E

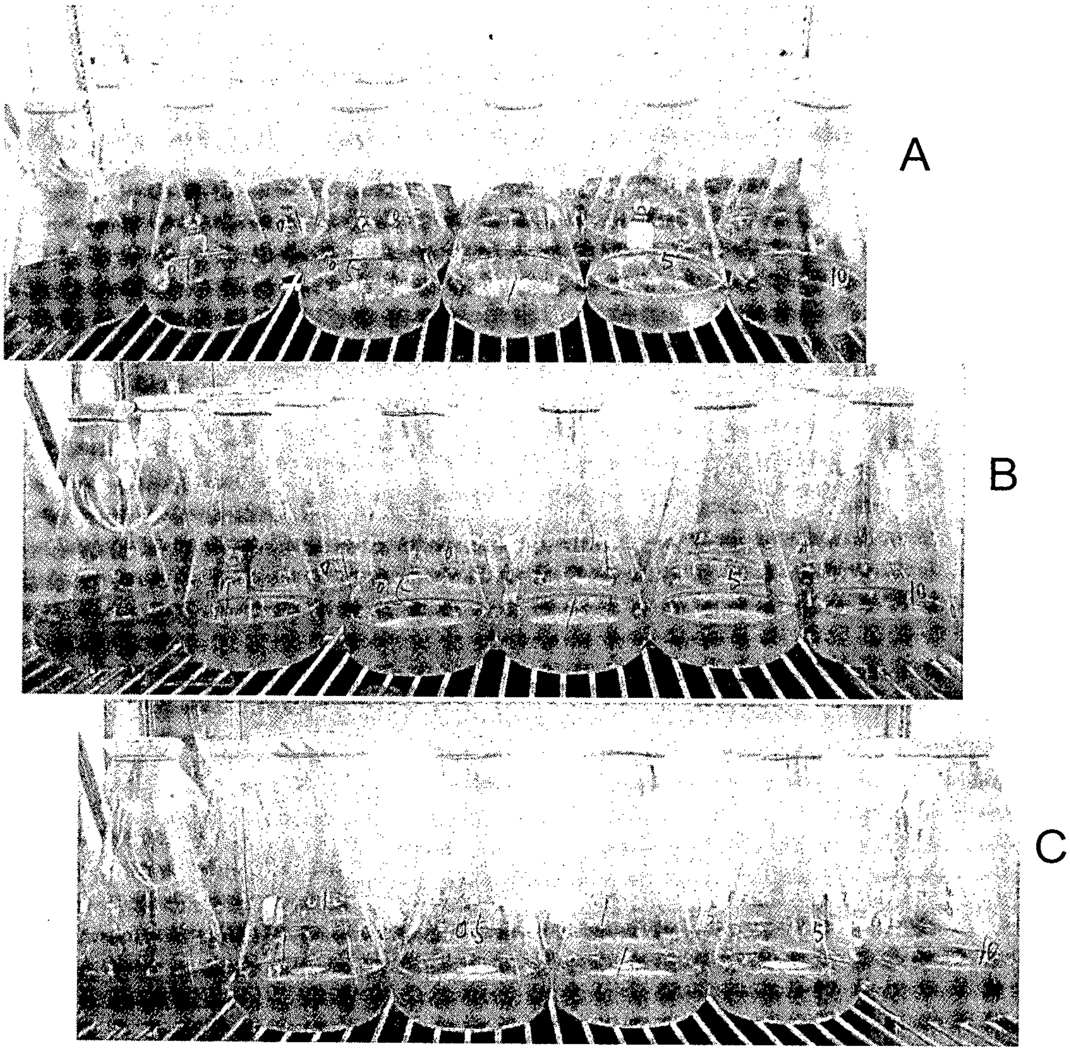


Fig. 5

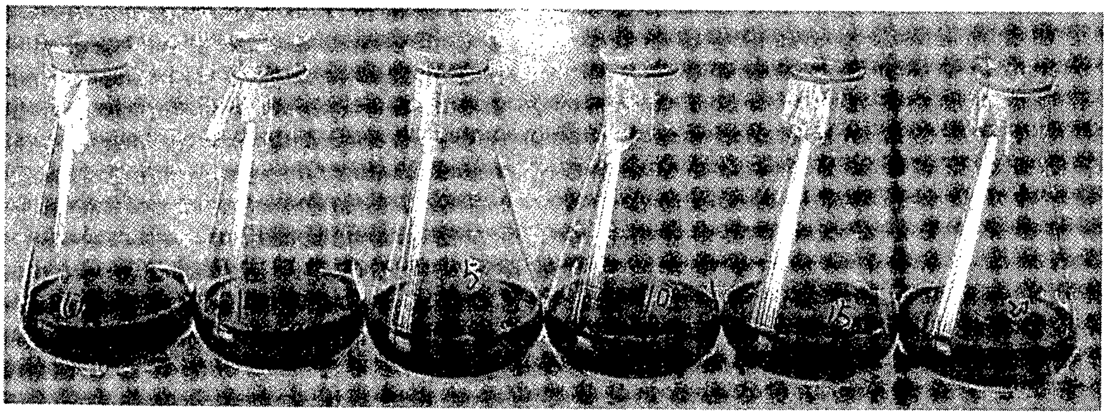

Fig. 6

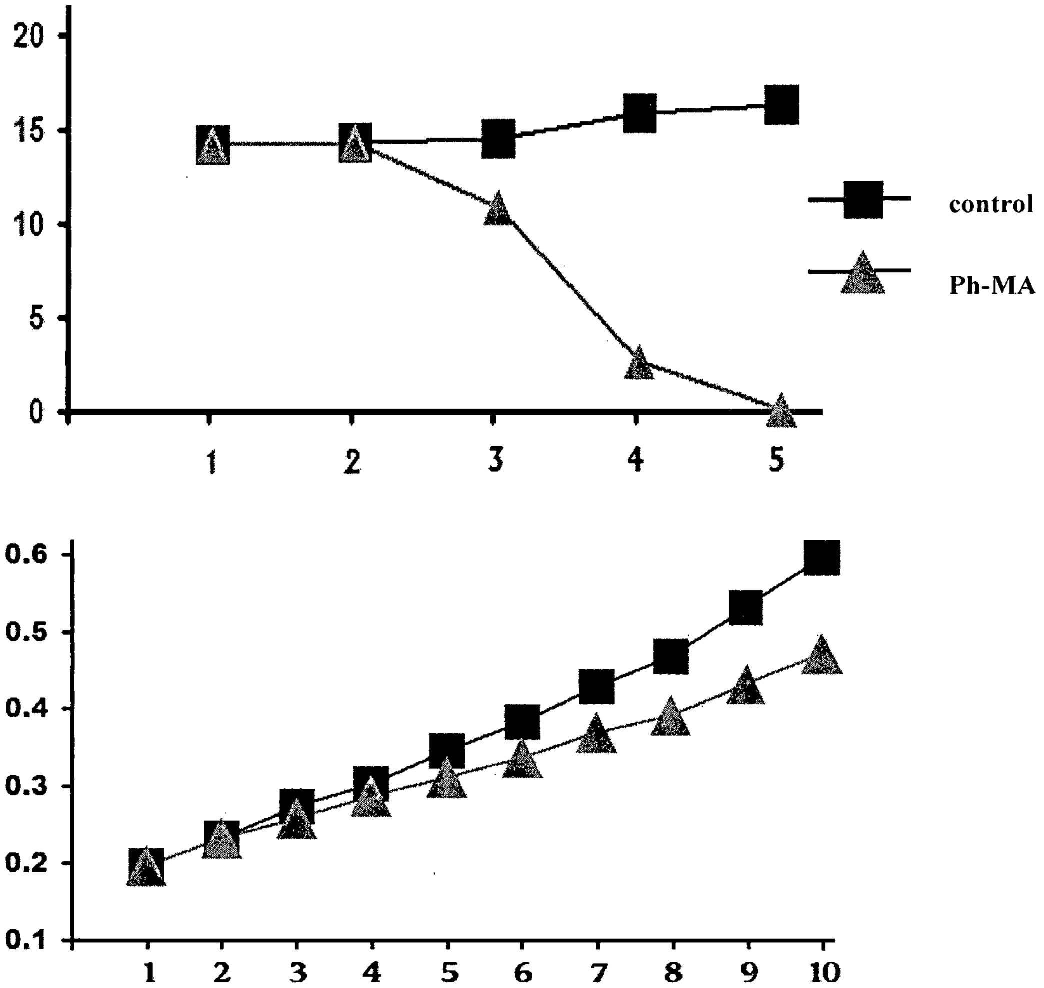


Fig. 7

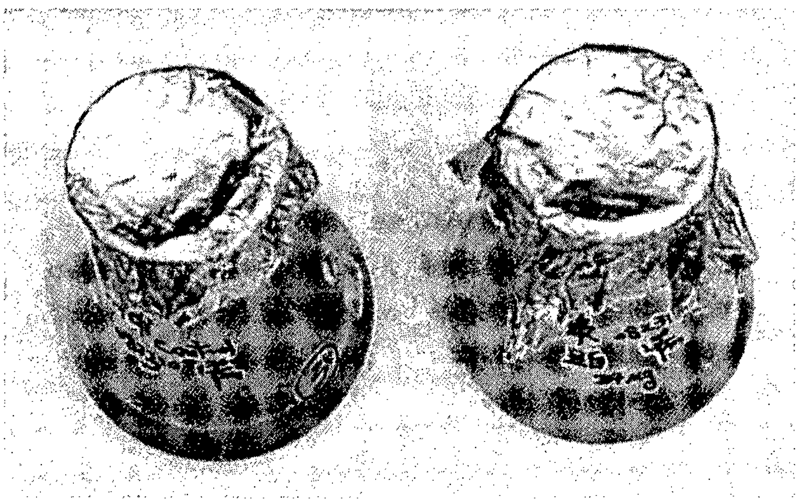

Fig. 8A

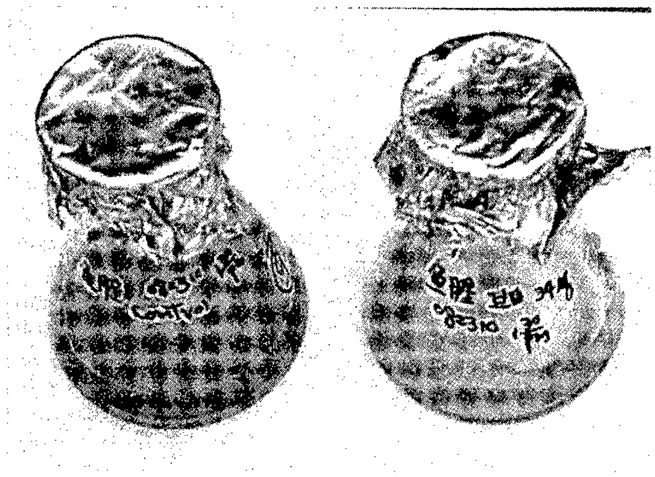

Fig. 8B

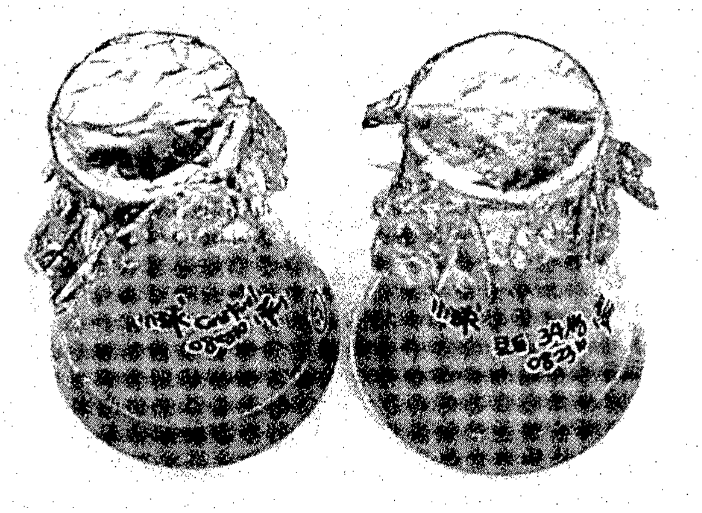

Fig. 8C

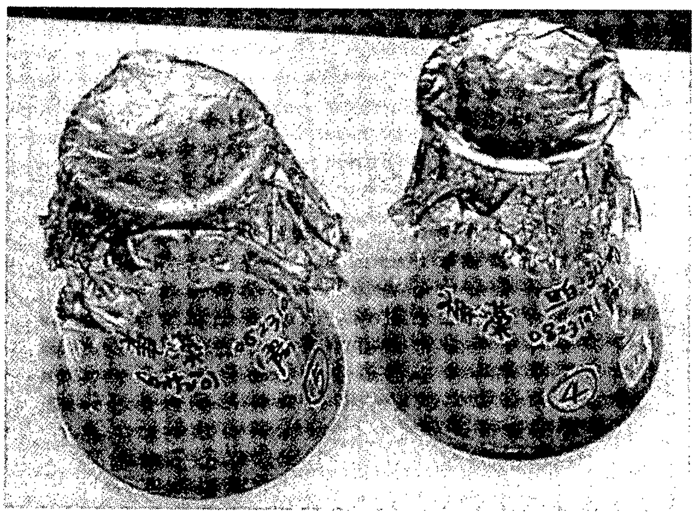

Fig. 8D

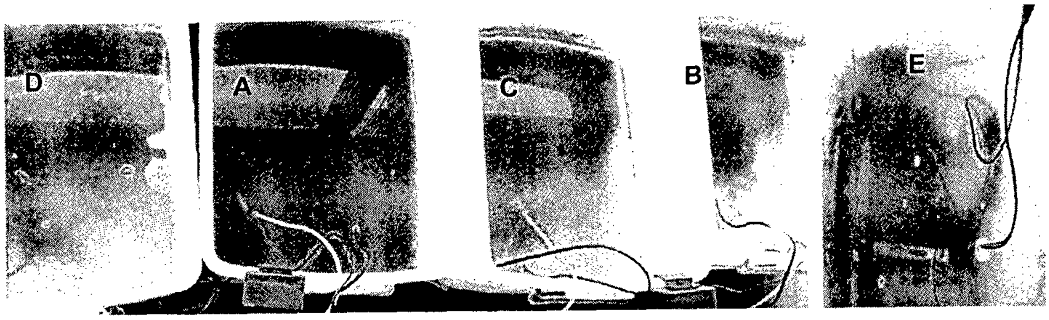


Fig. 9

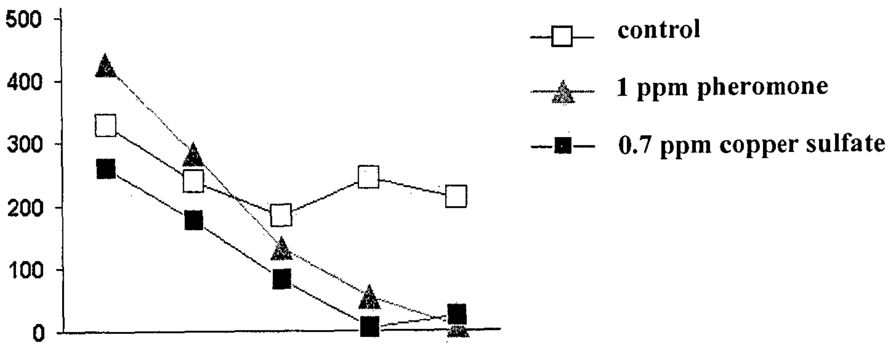


Fig. 10A

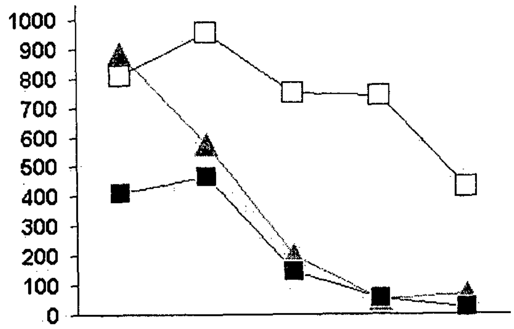


Fig. 10B

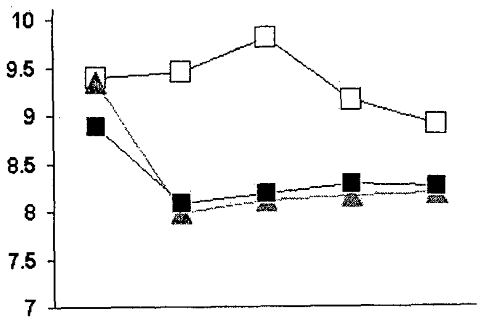


Fig. 10C

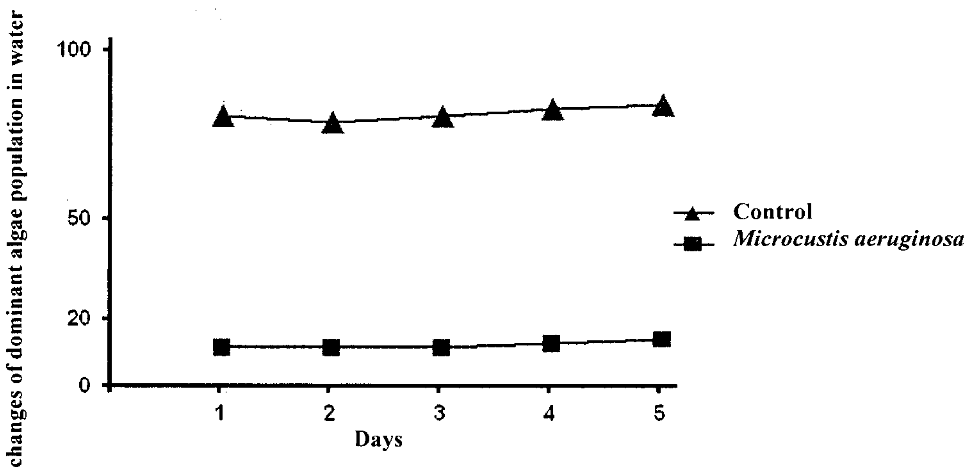


Fig. 10D-1

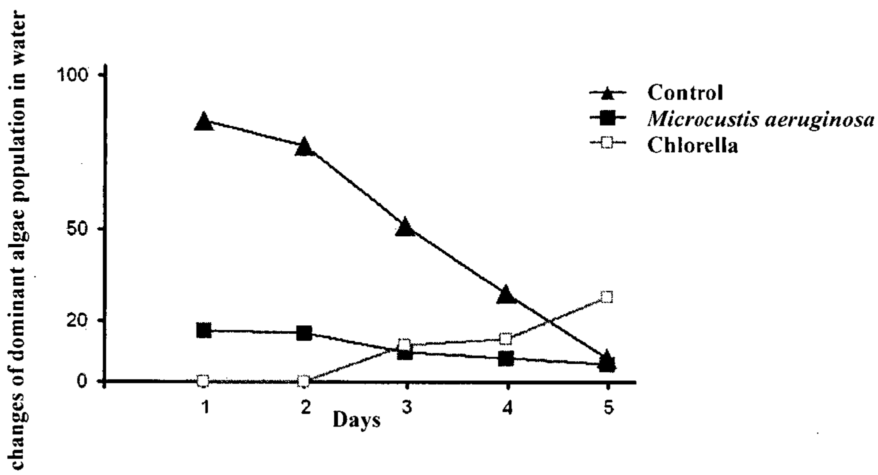


Fig. 10D-2

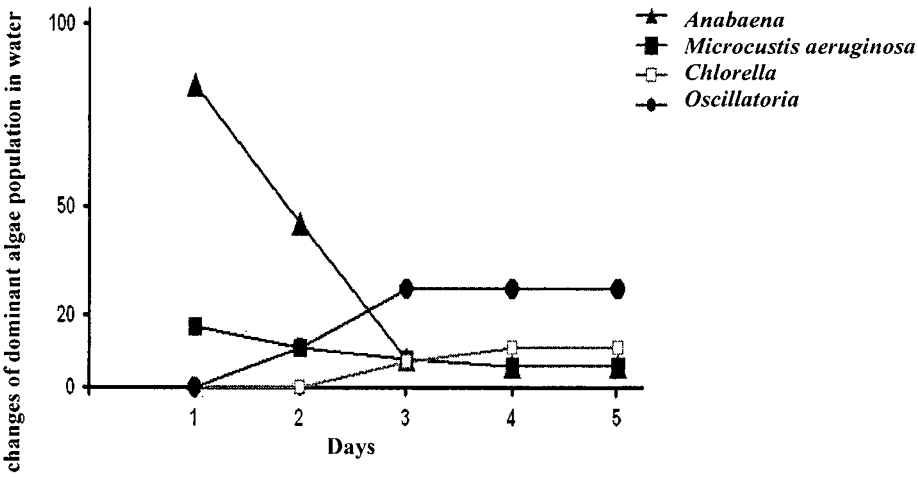


Fig. 10D-3

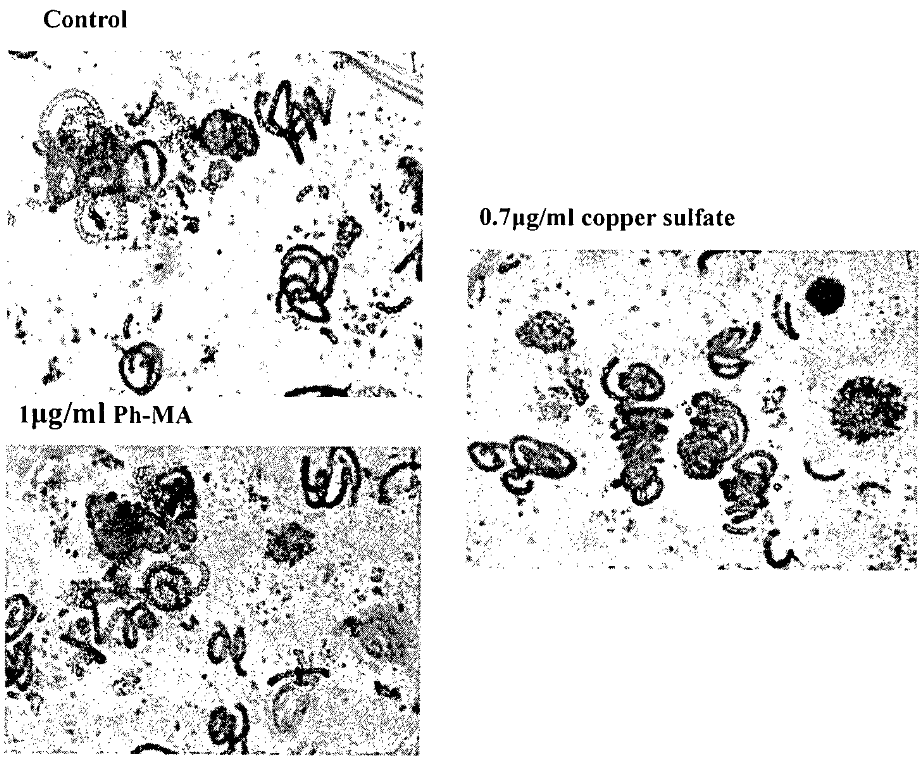


Fig. 11A

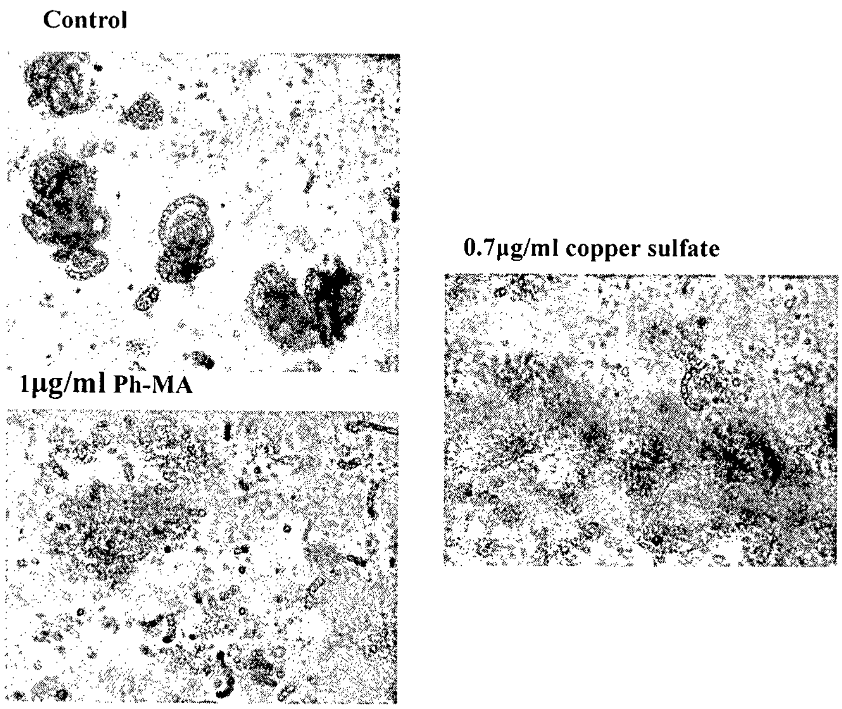


Fig. 11B

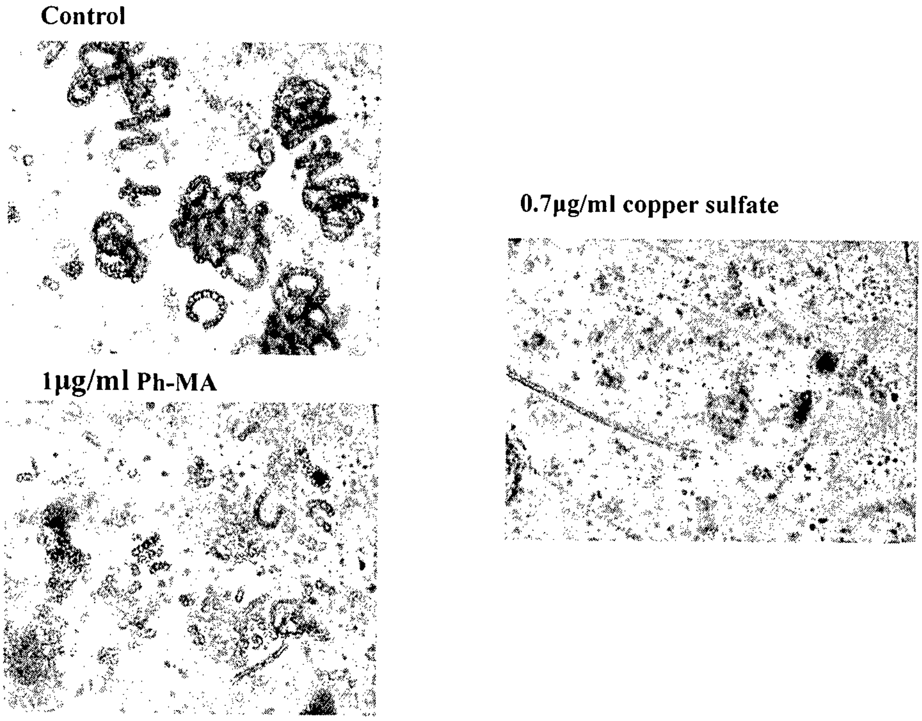


Fig. 11C

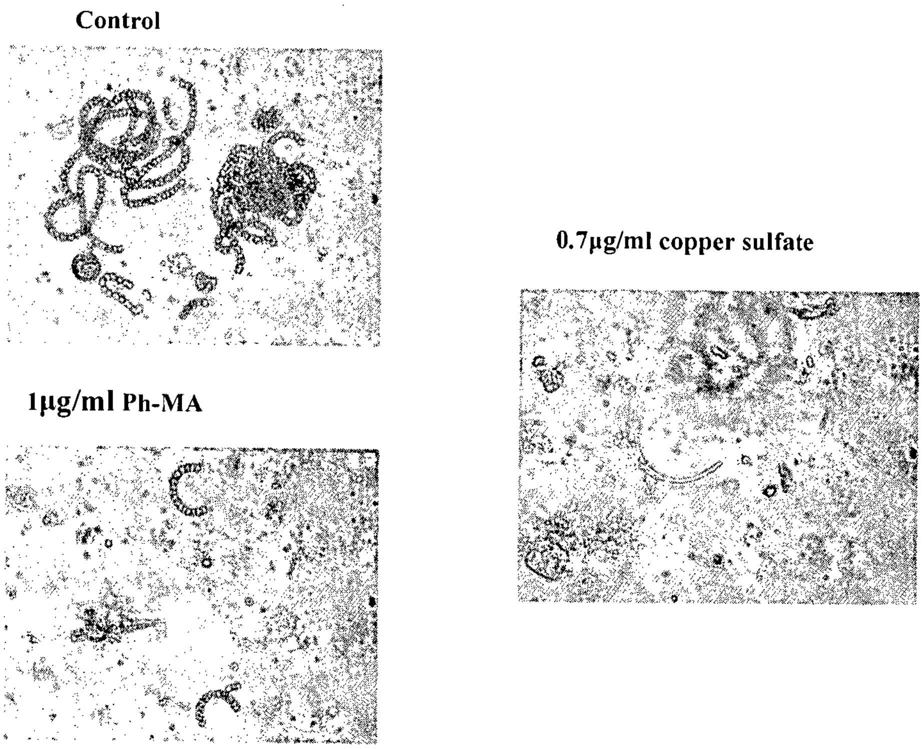


Fig. 11D

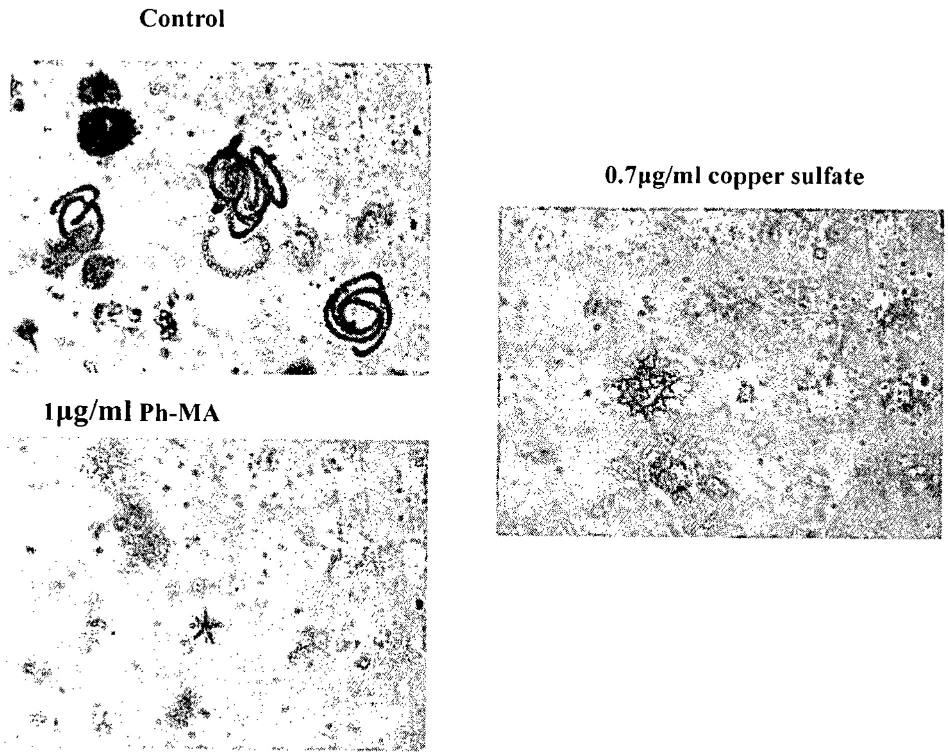


Fig. 11E

# INTERNATIONAL SEARCH REPORT

International application No.
**PCT/CN2012/073120**

**A. CLASSIFICATION OF SUBJECT MATTER**

See the extra sheet

According to International Patent Classification (IPC) or to both national classification and IPC

B. FIELDS SEARCHED

Minimum documentation searched (classification system followed by classification symbols)

IPC：C12N, C07K, C02F, C12R

Documentation searched other than minimum documentation to the extent that such documents are included in the fields searched

Electronic data base consulted during the international search (name of data base and, where practicable, search terms used)

DATABASE: CPRSABS, CNABS, CJFD, CSCD, SIPONPL, CNKI, DWPI, SIPOABS, CPEA, ISI WEB OF KNOWLEDGE, CA, BA, MEDLINE, PUBMED, GENBANK+EMBL+DDBJ

KEYWORDS: CGMCC 478, cyanophyta, cyanobacteria, blue algae, blue green algae, blue-green algae, blue green bacteria, blue-green bacteria, blue bacteria, hybridoma, chroococaceae, chroococaceaeus, oscillatoria, nostoc, fractionation, microcystis, anabaena, chlorella, scenedesmus, monoclonal antibody, antibody, antibodies, antigen, sequence search on the sequences of SEQ ID NOs：2、3、6、7

C. DOCUMENTS CONSIDERED TO BE RELEVANT

| Category* | Citation of document, with indication, where appropriate, of the relevant passages | Relevant to claim No. |
|---|---|---|
| X | LIU, Zhongzhu et al., "Preparing Monoclonal Antibodies against *Anabaena azollae* and Applied Research", SCIENCE IN CHINA, SER.B，31 Jan.1989(31.01.1989), no.1, pages 44-52, ISSN:1674-7224, see abstract, materials and methods, results | 1-3,5 |
| X | LIU, Zhongzhu et al., "Studies on Monoclonal Antibodies against Anabaena azollae", FUJIAN JOURNAL OF AGRICULTURAL ACIENCES, 31 DEC.1986(31.12.1986), vol.1, no.2, pages 1-7, ISSN: 1008-0384, see the abstract, materials and methods, results | 1-3,5 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 14 June 2012(14.06.2012) | 28 June 2012(28.06.2012) |

Name and mailing address of the ISA
State Intellectual Property Office of the P. R. China
No. 6, Xitucheng Road, Jimenqiao
Haidian District, Beijing 100088, China
Facsimile No. (86-10)62019451

Authorized officer
YU, Na
Telephone No. (86-10)62411084

Form PCT/ISA /210 (second sheet) (July 2009)

# INTERNATIONAL SEARCH REPORT

International application No.
**PCT/CN2012/073120**

C (Continuation). DOCUMENTS CONSIDERED TO BE RELEVANT

| Category* | Citation of document, with indication, where appropriate, of the relevant passages | Relevant to claim No. |
|---|---|---|
| A | D.S. RENNIS, "Differences in antigenic reactivity of six types of phycoerythrin to monoclonal anti-R-phycoerythrin", Phycologia, 31 July 1991(31.07.1991), Vol.30, No.4, pages 329-338, ISSN: 0031-8884, see the whole document | 1-13 |
| A | CN101429507A (ATOMIC ENERGY USE INST CHINESE ACAD AGRIC SCI), 13 May 2009(13.05.2009), see the whole document | 1-13 |
| A | KR20040097406A (BODITECHMED INC), 18 Nov. 2004(18.11.2004), see the whole document | 1-13 |
| A | JP7089998A (MITSUBISHI YUKA BCL KK), 04 April 1995(04.04.1995) , see the whole document | 1-13 |
| A | SONG, Limin et al., "Construction, expression and identification of scFv against microcystin-LR", CHINESE HIGH TECHNOLOGY LETTERS, 31 March 2010(31.03.2010), Vol.20, No.3, pages327-330, ISSN: 1002-0470, see the whole document | 1-13 |

Form PCT/ISA /210 (continuation of second sheet ) (July 2009)

INTERNATIONAL SEARCH REPORT

International application No.
PCT/CN2012/073120

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA /210 (continuation of first sheet (2)) (July 2009)

**INTERNATIONAL SEARCH REPORT**
Information on patent family members

International application No.
**PCT/CN2012/073120**

| Patent Documents referred in the Report | Publication Date | Patent Family | Publication Date |
|---|---|---|---|
| CN101429507A | 13.05.2009 | NONE | |
| KR20040097406A | 18.11.2004 | NONE | |
| JP7089998A | 04.04.1995 | NONE | |

Form PCT/ISA /210 (patent family annex) (July 2009)

## INTERNATIONAL SEARCH REPORT

International application No.
**PCT/CN2012/073120**

### A. CLASSIFICATION OF SUBJECT MATTER

C12N 5/12 (2006.01) i
C07K 16/14 (2006.01) i
C07K 19/00 (2006.01) i
C12N 15/13 (2006.01) i
C12N 15/62 (2006.01) i
C12N 15/63 (2006.01) i
C12N 15/70 (2006.01) i
C02F 1/50 (2006.01) i
C12R 1/89 (2006.01) n

### Box No. III Observations where unity of invention is lacking:

This International Searching Authority found two groups of inventions in this international application, as follows:

1. claims: 1-8, 10 and 12-13

   related to a hybridoma cell deposited as CGMCC No.4783 and the monoclonal antibodies secreted by the hybridoma cell; a mimetic antibody polypeptide which has an ability to recognize and bind to blue algae and its encoding gene, wherein the said mimetic antibody polypeptide is the antigen-binding fragment of the said monoclonal antibody, or the small molecular polypeptide designed according to the functional region of the said antigen-binding fragment; the recombinant antibody polypeptide against blue algae, its encoding gene, the preparing method thereof and the application, wherein the said recombinant antibody polypeptide against blue algae is constructed by the above mimetic antibody polypeptide having an ability to recognize and bind to blue algae operably linked to the carboxyl terminus of colicine polypeptide;

2. claims: 9 and 11

   related to the gene encoding the recombinant antibody polypeptide against blue algae, and the recombinant expression vector comprising the gene encoding the recombinant antibody polypeptide against blue algae;

The recombinant antibody polypeptides against blue algae of groups 1 and 2 are different, and do not have same structural unit between them. That is to say, groups 1 and 2 do not have the same or corresponding special technical feature, and do not linked by a single general inventive concept. Therefore, they do not meet the requirement of unity of invention in accordance with Rules 13.1, 13.2 and 13.3.

Form PCT/ISA /210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 200910092128

SEQUENCE LISTING

<110> Protein Design Lab, Ltd.

<120>ANTI-CYANOBACTERIA RECOMBINANT ANTIBODY POLYPEPTIDE, GENE THEREOF, AND PREPARATION METHOD THEREOF

<130> PC11853/JJQ

<160> 9

<170> PatentIn version 3.3

<210> 1

<211> 1878

<212> DNA

<213> Nucleotide sequence encoding colicin Ia

<400> 1

atgtctgacc ctgtacgtat tacaaatccc ggtgcagaat cgctggggta tgattcagat 60

ggccatgaaa ttatggccgt tgatatttat gtaaaccctc cacgtgtcga tgtctttcat 120

ggtaccccgc ctgcatggag ttccttcggg aacaaaacca tctggggcgg aaacgagtgg 180

gttgatgatt ccccaacccg aagtgatatc gaaaaaaggg acaaggaaat cacagcgtac 240

aaaaacacgc tcagcgcgca gcagaaagag aatgagaata agcgtactga agccggaaaa 300

cgcctctctg cggcgattgc tgcaagggaa aaagatgaaa acacactgaa aacactccgt 360

gccggaaacg cagatgccgc tgatattaca cgacaggagt tcagactcct gcaggcagag 420

ctgagagaat acggattccg tactgaaatc gccggatatg acgccctccg gctgcataca 480

gagagccgga tgctgtttgc tgatgctgat tctcttcgta tatctccccg ggaggccagg 540

tcgttaatcg aacaggctga aaaacggcag aaggatgcgc agaacgcaga caagaaggcc 600

gctgatatgc ttgctgaata cgagcgcaga aaaggtattc tggacacccg gttgtcagag 660

ctggaaaaaa atggcggggc agcccttgcc gttcttgatg cacaacaggc ccgtctgctc 720

gggcagcaga cacggaatga cagggccatt tcagaggccc ggaataaact cagttcagtg 780

acggaatcgc ttaacacggc ccgtaatgca ttaaccagag ctgaacaaca gctgacgcaa 840

cagaaaaaca cgcctgacgg caaaacgata gtttcccctg aaaaattccc ggggcgttca 900

tcaacaaatc attctattgt tgtgagcggt gatccgagat ttgccggtac gataaaaatc 960

acaaccagcg cagtcatcga taaccgtgca aacctgaatt atcttctgag ccattccggt 1020

ctggactata aacgcaatat tctgaatgac cggaatccgg tggtgacaga ggatgtggaa 1080
ggtgacaaga aaatttataa tgctgaagtt gctgaatggg ataagttacg gcaaagattg 1140
cttgatgcca gaaataaaat cacctctgct gaatctgcgg taaattcggc gagaaataac 1200
ctcagtgcca gaacaaatga gcaaaagcat gcaaatgacg ctcttaatgc cctgttgaag 1260
gaaaaagaga atatacgtaa ccagctttcc ggcatcaatc agaagatagc ggaagagaaa 1320
agaaaacagg atgaactgaa ggcaacgaaa gacgcaatta atttcacaac agagttcctg 1380
aaatcagttt cagaaaaata tggtgcaaaa gctgagcagt tagccagaga gatggccggg 1440
caggctaaag ggaagaaaat acgtaatgtt gaagaggcat taaaaacgta tgaaaagtac 1500
cgggctgaca ttaacaaaaa aattaatgca aaagatcgtg cagcgattgc cgcagccctt 1560
gagtctgtga agctgtctga tatatcgtct aatctgaaca gattcagtcg gggactggga 1620
tatgcaggaa aatttacaag tcttgctgac tggatcactg agtttggtaa ggctgtccgg 1680
acagagaact ggcgtcctct ttttgttaaa acagaaacca tcatagcagg caatgccgca 1740
acggctcttg tggcactggt cttcagtatt cttaccggaa gcgctttagg cattatcggg 1800
tatggtttac tgatggctgt caccggtgcg ctgattgatg aatcgcttgt ggaaaaagcg 1860
aataagttctggggtatt 1878

<210> 2
<211> 87
<212> DNA
<213> Gene of antibody mimetics against *Microcystis aeruginosa* surface antigen
<400> 2

tcttattgga tgcagtgggt taagcaaaga ccggggcaag ggctggagtg gattggccag 60
cagtattggt ctacgccgcc gtggacg 87

<210> 3
<211> 29
<212> PRT
<213> Amino acid sequence of antibody mimetics against *Microcystis aeruginosa* surface antigen

<400> 3

Ser Tyr Trp Met Gln Trp Val Lys Gln Arg Pro Gly Gln Gly Leu Glu
1 5 10 15
Trp Ile Gly Gln Gln Tyr Trp Ser Thr Pro Pro Trp Thr
20 25

<210> 4
<211> 87
<212> DNA
<213> VHCDR1 N-C sequence in reversed order of gene of antibody mimetics against *Microcystis aeruginosa* surface antigen

<400> 4
cagatgtggt attcttgggt taagcaaaga ccggggcaag ggctggagtg gattggccag 60
cagtattggt ctacgccgcc gtggacg 87

<210> 5
<211> 29
<212> PRT
<213> VHCDR1 N-C sequence in reversed order of amino acid sequence of antibody mimetics against *Microcystis aeruginosa* surface antigen

<400> 5
Gln Met Trp Tyr Ser Trp Val Lys Gln Arg Pro Gly Gln Gly Leu Glu
1 5 10 15
Trp Ile Gly Gln Gln Tyr Trp Ser Thr Pro Pro Trp Thr
20 25

<210> 6
<211> 1965
<212> DNA
<213> Nucleotide sequence encoding anti-cyanobacteria polypeptide1

<400> 6

atgtctgacc ctgtacgtat tacaaatccc ggtgcagaat cgctggggta tgattcagat 60
ggccatgaaa ttatggccgt tgatatttat gtaaaccctc cacgtgtcga tgtctttcat 120
ggtaccccgc ctgcatggag ttccttcggg aacaaaacca tctggggcgg aaacgagtgg 180
gttgatgatt ccccaacccg aagtgatatc gaaaaaaggg acaaggaaat cacagcgtac 240
aaaaacacgc tcagcgcgca gcagaaagag aatgagaata agcgtactga agccggaaaa 300
cgcctctctg cggcgattgc tgcaagggaa aaagatgaaa acacactgaa aacactccgt 360
gccggaaacg cagatgccgc tgatattaca cgacaggagt tcagactcct gcaggcagag 420
ctgagagaat acggattccg tactgaaatc gccggatatg acgccctccg gctgcataca 480
gagagccgga tgctgtttgc tgatgctgat tctcttcgta tatctccccg ggaggccagg 540
tcgttaatcg aacaggctga aaaacggcag aaggatgcgc agaacgcaga caagaaggcc 600
gctgatatgc ttgctgaata cgagcgcaga aaaggtattc tggacacccg gttgtcagag 660
ctggaaaaaa atggcggggc agcccttgcc gttcttgatg cacaacaggc ccgtctgctc 720
gggcagcaga cacggaatga cagggccatt tcagaggccc ggaataaact cagttcagtg 780
acggaatcgc ttaacacggc ccgtaatgca ttaaccagag ctgaacaaca gctgacgcaa 840
cagaaaaaca cgcctgacgg caaaacgata gtttcccctg aaaaattccc ggggcgttca 900
tcaacaaatc attctattgt tgtgagcggt gatccgagat ttgccggtac gataaaaatc 960
acaaccagcg cagtcatcga taaccgtgca aacctgaatt atcttctgag ccattccggt 1020
ctggactata aacgcaatat tctgaatgac cggaatccgg tggtgacaga ggatgtggaa 1080
ggtgacaaga aaatttataa tgctgaagtt gctgaatggg ataagttacg gcaaagattg 1140
cttgatgcca gaaataaaat cacctctgct gaatctgcgg taaattcggc gagaaataac 1200
ctcagtgcca gaacaaatga gcaaaagcat gcaaatgacg ctcttaatgc cctgttgaag 1260
gaaaaagaga atatacgtaa ccagctttcc ggcatcaatc agaagatagc ggaagagaaa 1320
agaaaacagg atgaactgaa ggcaacgaaa gacgcaatta atttcacaac agagttcctg 1380
aaatcagttt cagaaaaata tggtgcaaaa gctgagcagt tagccagaga gatggccggg 1440
caggctaaag ggaagaaaat acgtaatgtt gaagaggcat taaaaacgta tgaaaagtac 1500
cgggctgaca ttaacaaaaa aattaatgca aaagatcgtg cagcgattgc cgcagccctt 1560
gagtctgtga agctgtctga tatatcgtct aatctgaaca gattcagtcg gggactggga 1620
tatgcaggaa aatttacaag tcttgctgac tggatcactg agtttggtaa ggctgtccgg 1680
acagagaact ggcgtcctct ttttgttaaa acagaaacca tcatagcagg caatgccgca 1740
acggctcttg tggcactggt cttcagtatt cttaccggaa gcgctttagg cattatcggg 1800

tatggtttac tgatggctgt caccggtgcg ctgattgatg aatcgcttgt ggaaaaagcg 1860

aataagttct ggggtatttc ttattggatg cagtgggtta agcaaagacc ggggcaaggg 1920

ctggagtgga ttggccagca gtattggtct acgccgccgt ggacg 1965

<210> 7

<211> 654

<212> PRT

<213> Amino acid sequence of anti-cyanobacteria polypeptide 1

<400> 7

Ser Asp Pro Val Arg Ile Thr Asn Pro Gly Ala Glu Ser Leu Gly Tyr
1 5 10 15

Asp Ser Asp Gly His Glu Ile Met Ala Val Asp Ile Tyr Val Asn Pro
20 25 30

Pro Arg Val Asp Val Phe His Gly Thr Pro Pro Ala Trp Ser Ser Phe
35 40 45

Gly Asn Lys Thr Ile Trp Gly Gly Asn Glu Trp Val Asp Asp Ser Pro
50 55 60

Thr Arg Ser Asp Ile Glu Lys Arg Asp Lys Glu Ile Thr Ala Tyr Lys
65 70 75 80

Asn Thr Leu Ser Ala Gln Gln Lys Glu Asn Glu Asn Lys Arg Thr Glu
85 90 95

Ala Gly Lys Arg Leu Ser Ala Ala Ile Ala Ala Arg Glu Lys Asp Glu
100 105 110

Asn Thr Leu Lys Thr Leu Arg Ala Gly Asn Ala Asp Ala Ala Asp Ile
115 120 125

Thr Arg Gln Glu Phe Arg Leu Leu Gln Ala Glu Leu Arg Glu Tyr Gly
130 135 140

Phe Arg Thr Glu Ile Ala Gly Tyr Asp Ala Leu Arg Leu His Thr Glu
145 150 155 160

Ser Arg Met Leu Phe Ala Asp Ala Asp Ser Leu Arg Ile Ser Pro Arg

165 170 175

Glu Ala Arg Ser Leu Ile Glu Gln Ala Glu Lys Arg Gln Lys Asp Ala

180 185 190

Gln Asn Ala Asp Lys Lys Ala Ala Asp Met Leu Ala Glu Tyr Glu Arg

195 200 205

Arg Lys Gly Ile Leu Asp Thr Arg Leu Ser Glu Leu Glu Lys Asn Gly

210 215 220

Gly Ala Ala Leu Ala Val Leu Asp Ala Gln Gln Ala Arg Leu Leu Gly

225 230 235 240

Gln Gln Thr Arg Asn Asp Arg Ala Ile Ser Glu Ala Arg Asn Lys Leu

245 250 255

Ser Ser Val Thr Glu Ser Leu Asn Thr Ala Arg Asn Ala Leu Thr Arg

260 265 270

Ala Glu Gln Gln Leu Thr Gln Gln Lys Asn Thr Pro Asp Gly Lys Thr

275 280 285

Ile Val Ser Pro Glu Lys Phe Pro Gly Arg Ser Ser Thr Asn His Ser

290 295 300

Ile Val Val Ser Gly Asp Pro Arg Phe Ala Gly Thr Ile Lys Ile Thr

305 310 315 320

Thr Ser Ala Val Ile Asp Asn Arg Ala Asn Leu Asn Tyr Leu Leu Ser

325 330 335

His Ser Gly Leu Asp Tyr Lys Arg Asn Ile Leu Asn Asp Arg Asn Pro

340 345 350

Val Val Thr Glu Asp Val Glu Gly Asp Lys Lys Ile Tyr Asn Ala Glu

355 360 365

Val Ala Glu Trp Asp Lys Leu Arg Gln Arg Leu Leu Asp Ala Arg Asn

370 375 380

Lys Ile Thr Ser Ala Glu Ser Ala Val Asn Ser Ala Arg Asn Asn Leu

385 390 395 400

Ser Ala Arg Thr Asn Glu Gln Lys His Ala Asn Asp Ala Leu Asn Ala

405 410 415

Leu Leu Lys Glu Lys Glu Asn Ile Arg Asn Gln Leu Ser Gly Ile Asn

420 425 430

Gln Lys Ile Ala Glu Glu Lys Arg Lys Gln Asp Glu Leu Lys Ala Thr

435 440 445

Lys Asp Ala Ile Asn Phe Thr Thr Glu Phe Leu Lys Ser Val Ser Glu

450 455 460

Lys Tyr Gly Ala Lys Ala Glu Gln Leu Ala Arg Glu Met Ala Gly Gln

465 470 475 480

Ala Lys Gly Lys Lys Ile Arg Asn Val Glu Glu Ala Leu Lys Thr Tyr

485 490 495

Glu Lys Tyr Arg Ala Asp Ile Asn Lys Lys Ile Asn Ala Lys Asp Arg

500 505 510

Ala Ala Ile Ala Ala Ala Leu Glu Ser Val Lys Leu Ser Asp Ile Ser

515 520 525

Ser Asn Leu Asn Arg Phe Ser Arg Gly Leu Gly Tyr Ala Gly Lys Phe

530 535 540

Thr Ser Leu Ala Asp Trp Ile Thr Glu Phe Gly Lys Ala Val Arg Thr

545 550 555 560

Glu Asn Trp Arg Pro Leu Phe Val Lys Thr Glu Thr Ile Ile Ala Gly

565 570 575

Asn Ala Ala Thr Ala Leu Val Ala Leu Val Phe Ser Ile Leu Thr Gly

580 585 590

Ser Ala Leu Gly Ile Ile Gly Tyr Gly Leu Leu Met Ala Val Thr Gly

595 600 605

Ala Leu Ile Asp Glu Ser Leu Val Glu Lys Ala Asn Lys Phe Trp Gly

610 615 620

Ile Ser Tyr Trp Met Gln Trp Val Lys Gln Arg Pro Gly Gln Gly Leu

625 630 635 640

Glu Trp Ile Gly Gln Gln Tyr Trp Ser Thr Pro Pro Trp Thr

645 650

<210> 8

<211> 1965

<212> DNA

<213> Nucleotide sequence encoding antifugal polypeptide 2

<400> 8

atgtctgacc ctgtacgtat tacaaatccc ggtgcagaat cgctggggta tgattcagat 60
ggccatgaaa ttatggccgt tgatatttat gtaaaccctc cacgtgtcga tgtctttcat 120
ggtaccccgc ctgcatggag ttccttcggg aacaaaacca tctggggcgg aaacgagtgg 180
gttgatgatt ccccaacccg aagtgatatc gaaaaaaggg acaaggaaat cacagcgtac 240
aaaaacacgc tcagcgcgca gcagaaagag aatgagaata agcgtactga agccggaaaa 300
cgcctctctg cggcgattgc tgcaagggaa aaagatgaaa acacactgaa aacactccgt 360
gccggaaacg cagatgccgc tgatattaca cgacaggagt tcagactcct gcaggcagag 420
ctgagagaat acggattccg tactgaaatc gccggatatg acgccctccg gctgcataca 480
gagagccgga tgctgtttgc tgatgctgat tctcttcgta tatctccccg ggaggccagg 540
tcgttaatcg aacaggctga aaaacggcag aaggatgcgc agaacgcaga caagaaggcc 600
gctgatatgc ttgctgaata cgagcgcaga aaaggtattc tggacacccg gttgtcagag 660
ctggaaaaaa atggcggggc agcccttgcc gttcttgatg cacaacaggc ccgtctgctc 720
gggcagcaga cacggaatga cagggccatt tcagaggccc ggaataaact cagttcagtg 780
acggaatcgc ttaacacggc ccgtaatgca ttaaccagag ctgaacaaca gctgacgcaa 840
cagaaaaaca cgcctgacgg caaaacgata gtttcccctg aaaaattccc ggggcgttca 900
tcaacaaatc attctattgt tgtgagcggt gatccgagat ttgccggtac gataaaaatc 960
acaaccagcg cagtcatcga taaccgtgca aacctgaatt atcttctgag ccattccggt 1020
ctggactata aacgcaatat tctgaatgac cggaatccgg tggtgacaga ggatgtggaa 1080
ggtgacaaga aaatttataa tgctgaagtt gctgaatggg ataagttacg gcaaagattg 1140
cttgatgcca gaaataaaat cacctctgct gaatctgcgg taaattcggc gagaaataac 1200
ctcagtgcca gaacaaatga gcaaaagcat gcaaatgacg ctcttaatgc cctgttgaag 1260
gaaaaagaga atatacgtaa ccagctttcc ggcatcaatc agaagatagc ggaagagaaa 1320
agaaaacagg atgaactgaa ggcaacgaaa gacgcaatta atttcacaac agagttcctg 1380

aaatcagttt cagaaaaata tggtgcaaaa gctgagcagt tagccagaga gatggccggg 1440

caggctaaag ggaagaaaat acgtaatgtt gaagaggcat taaaaacgta tgaaaagtac 1500

cgggctgaca ttaacaaaaa aattaatgca aaagatcgtg cagcgattgc cgcagccctt 1560

gagtctgtga agctgtctga tatatcgtct aatctgaaca gattcagtcg gggactggga 1620

tatgcaggaa aatttacaag tcttgctgac tggatcactg agtttggtaa ggctgtccgg 1680

acagagaact ggcgtcctct ttttgttaaa acagaaacca tcatagcagg caatgccgca 1740

acggctcttg tggcactggt cttcagtatt cttaccggaa gcgctttagg cattatcggg 1800

tatggtttac tgatggctgt caccggtgcg ctgattgatg aatcgcttgt ggaaaaagcg 1860

aataagttct ggggtattca gatgtggtat tcttgggtta agcaaagacc ggggcaaggg 1920

ctggagtgga ttggccagca gtattggtct acgccgccgt ggacg 1965

<210> 9

<211> 654

<212> PRT

<213> Amino acid sequence of anti-cyanobacteria polypeptide 2

<400> 9

Ser Asp Pro Val Arg Ile Thr Asn Pro Gly Ala Glu Ser Leu Gly Tyr

1 5 10 15

Asp Ser Asp Gly His Glu Ile Met Ala Val Asp Ile Tyr Val Asn Pro

20 25 30

Pro Arg Val Asp Val Phe His Gly Thr Pro Pro Ala Trp Ser Ser Phe

35 40 45

Gly Asn Lys Thr Ile Trp Gly Gly Asn Glu Trp Val Asp Asp Ser Pro

50 55 60

Thr Arg Ser Asp Ile Glu Lys Arg Asp Lys Glu Ile Thr Ala Tyr Lys

65 70 75 80

Asn Thr Leu Ser Ala Gln Gln Lys Glu Asn Glu Asn Lys Arg Thr Glu

85 90 95

Ala Gly Lys Arg Leu Ser Ala Ala Ile Ala Ala Arg Glu Lys Asp Glu

100 105 110

Asn Thr Leu Lys Thr Leu Arg Ala Gly Asn Ala Asp Ala Ala Asp Ile

115 120 125

Thr Arg Gln Glu Phe Arg Leu Leu Gln Ala Glu Leu Arg Glu Tyr Gly

130 135 140

Phe Arg Thr Glu Ile Ala Gly Tyr Asp Ala Leu Arg Leu His Thr Glu

145 150 155 160

Ser Arg Met Leu Phe Ala Asp Ala Asp Ser Leu Arg Ile Ser Pro Arg

165 170 175

Glu Ala Arg Ser Leu Ile Glu Gln Ala Glu Lys Arg Gln Lys Asp Ala

180 185 190

Gln Asn Ala Asp Lys Lys Ala Ala Asp Met Leu Ala Glu Tyr Glu Arg

195 200 205

Arg Lys Gly Ile Leu Asp Thr Arg Leu Ser Glu Leu Glu Lys Asn Gly

210 215 220

Gly Ala Ala Leu Ala Val Leu Asp Ala Gln Gln Ala Arg Leu Leu Gly

225 230 235 240

Gln Gln Thr Arg Asn Asp Arg Ala Ile Ser Glu Ala Arg Asn Lys Leu

245 250 255

Ser Ser Val Thr Glu Ser Leu Asn Thr Ala Arg Asn Ala Leu Thr Arg

260 265 270

Ala Glu Gln Gln Leu Thr Gln Gln Lys Asn Thr Pro Asp Gly Lys Thr

275 280 285

Ile Val Ser Pro Glu Lys Phe Pro Gly Arg Ser Ser Thr Asn His Ser

290 295 300

Ile Val Val Ser Gly Asp Pro Arg Phe Ala Gly Thr Ile Lys Ile Thr

305 310 315 320

Thr Ser Ala Val Ile Asp Asn Arg Ala Asn Leu Asn Tyr Leu Leu Ser

325 330 335

His Ser Gly Leu Asp Tyr Lys Arg Asn Ile Leu Asn Asp Arg Asn Pro

340 345 350

Val Val Thr Glu Asp Val Glu Gly Asp Lys Lys Ile Tyr Asn Ala Glu

355 360 365

Val Ala Glu Trp Asp Lys Leu Arg Gln Arg Leu Leu Asp Ala Arg Asn

370 375 380

Lys Ile Thr Ser Ala Glu Ser Ala Val Asn Ser Ala Arg Asn Asn Leu

385 390 395 400

Ser Ala Arg Thr Asn Glu Gln Lys His Ala Asn Asp Ala Leu Asn Ala

405 410 415

Leu Leu Lys Glu Lys Glu Asn Ile Arg Asn Gln Leu Ser Gly Ile Asn

420 425 430

Gln Lys Ile Ala Glu Glu Lys Arg Lys Gln Asp Glu Leu Lys Ala Thr

435 440 445

Lys Asp Ala Ile Asn Phe Thr Thr Glu Phe Leu Lys Ser Val Ser Glu

450 455 460

Lys Tyr Gly Ala Lys Ala Glu Gln Leu Ala Arg Glu Met Ala Gly Gln

465 470 475 480

Ala Lys Gly Lys Lys Ile Arg Asn Val Glu Glu Ala Leu Lys Thr Tyr

485 490 495

Glu Lys Tyr Arg Ala Asp Ile Asn Lys Lys Ile Asn Ala Lys Asp Arg

500 505 510

Ala Ala Ile Ala Ala Ala Leu Glu Ser Val Lys Leu Ser Asp Ile Ser

515 520 525

Ser Asn Leu Asn Arg Phe Ser Arg Gly Leu Gly Tyr Ala Gly Lys Phe

530 535 540

Thr Ser Leu Ala Asp Trp Ile Thr Glu Phe Gly Lys Ala Val Arg Thr

545 550 555 560

Glu Asn Trp Arg Pro Leu Phe Val Lys Thr Glu Thr Ile Ile Ala Gly

565 570 575

Asn Ala Ala Thr Ala Leu Val Ala Leu Val Phe Ser Ile Leu Thr Gly

580 585 590

Ser Ala Leu Gly Ile Ile Gly Tyr Gly Leu Leu Met Ala Val Thr Gly

595 600 605

Ala Leu Ile Asp Glu Ser Leu Val Glu Lys Ala Asn Lys Phe Trp Gly

610 615 620

Ile Gln Met Trp Tyr Ser Trp Val Lys Gln Arg Pro Gly Gln Gly Leu

625 630 635 640

Glu Trp Ile Gly Gln Gln Tyr Trp Ser Thr Pro Pro Trp Thr

645 650

SEQUENCE LISTING

<110> Protein Design Lab, Ltd.

<120> ANTI-CYANOBACTERIA RECOMBINANT ANTIBODY POLYPEPTIDE, GENE THEREOF, AND PREPARATION METHOD THEREOF

<130> PC11853/JJQ

<160> 15

<170> PatentIn version 3.5

<210> 1
<211> 1878
<212> DNA
<213> Artificial Sequence

<220>
<223> Nucleotide sequence encoding colicin Ia

<400> 1
atgtctgacc ctgtacgtat tacaaatccc ggtgcagaat cgctggggta tgattcagat 60

ggccatgaaa ttatggccgt tgatatttat gtaaaccctc cacgtgtcga tgtctttcat 120

ggtaccccgc ctgcatggag ttccttcggg aacaaaacca tctggggcgg aaacgagtgg 180

gttgatgatt ccccaacccg aagtgatatc gaaaaaaggg acaaggaaat cacagcgtac 240

aaaaacacgc tcagcgcgca gcagaaagag aatgagaata agcgtactga agccggaaaa 300

cgcctctctg cggcgattgc tgcaagggaa aaagatgaaa acacactgaa aacactccgt 360

gccggaaacg cagatgccgc tgatattaca cgacaggagt tcagactcct gcaggcagag 420

ctgagagaat acggattccg tactgaaatc gccggatatg acgccctccg gctgcataca 480

gagagccgga tgctgtttgc tgatgctgat tctcttcgta tatctccccg ggaggccagg 540

tcgttaatcg aacaggctga aaaacggcag aaggatgcgc agaacgcaga caagaaggcc 600

gctgatatgc ttgctgaata cgagcgcaga aaaggtattc tggacacccg gttgtcagag 660

ctggaaaaaa atggcggggc agcccttgcc gttcttgatg cacaacaggc ccgtctgctc 720

gggcagcaga cacggaatga cagggccatt tcagaggccc ggaataaact cagttcagtg 780

acggaatcgc ttaacacggc ccgtaatgca ttaaccagag ctgaacaaca gctgacgcaa 840

cagaaaaaca cgcctgacgg caaaacgata gtttcccctg aaaaattccc ggggcgttca 900

tcaacaaatc attctattgt tgtgagcggt gatccgagat ttgccggtac gataaaaatc 960

acaaccagcg cagtcatcga taaccgtgca aacctgaatt atcttctgag ccattccggt 1020

ctggactata aacgcaatat tctgaatgac cggaatccgg tggtgacaga ggatgtggaa 1080

ggtgacaaga aaatttataa tgctgaagtt gctgaatggg ataagttacg gcaaagattg 1140

cttgatgcca gaaataaaat cacctctgct gaatctgcgg taaattcggc gagaaataac 1200

ctcagtgcca gaacaaatga gcaaaagcat gcaaatgacg ctcttaatgc cctgttgaag 1260

gaaaaagaga atatacgtaa ccagctttcc ggcatcaatc agaagatagc ggaagagaaa 1320

agaaaacagg atgaactgaa ggcaacgaaa gacgcaatta atttcacaac agagttcctg 1380

aaatcagttt cagaaaaata tggtgcaaaa gctgagcagt tagccagaga gatggccggg 1440

caggctaaag ggaagaaaat acgtaatgtt gaagaggcat taaaaacgta tgaaaagtac 1500

cgggctgaca ttaacaaaaa aattaatgca aaagatcgtg cagcgattgc cgcagccctt 1560

gagtctgtga agctgtctga tatatcgtct aatctgaaca gattcagtcg gggactggga 1620

tatgcaggaa aatttacaag tcttgctgac tggatcactg agtttggtaa ggctgtccgg 1680

acagagaact ggcgtcctct tttgttaaa acagaaacca tcatagcagg caatgccgca 1740

acggctcttg tggcactggt cttcagtatt cttaccggaa gcgctttagg cattatcggg 1800

tatggtttac tgatggctgt caccggtgcg ctgattgatg aatcgcttgt ggaaaaagcg 1860

aataagttct ggggtatt 1878

<210> 2
<211> 87
<212> DNA
<213> Artificial Sequence

<220>
<223> Gene of antibody mimetics against Microcystis aeruginosa surface antigen

<400> 2
tcttattgga tgcagtgggt taagcaaaga ccggggcaag ggctggagtg gattggccag 60

cagtattggt ctacgccgcc gtggacg 87

<210> 3
<211> 29
<212> PRT
<213> Artificial Sequence

<220>
<223> Amino acid sequence of antibody mimetics against Microcystis aeruginosa surface antigen

<400> 3

Ser Tyr Trp Met Gln Trp Val Lys Gln Arg Pro Gly Gln Gly Leu Glu
1               5                   10                  15

Trp Ile Gly Gln Gln Tyr Trp Ser Thr Pro Pro Trp Thr
            20                  25

<210> 4
<211> 87
<212> DNA
<213> Artificial Sequence

<220>
<223> VHCDR1 N-C sequence in reversed order of gene of antibody mimetics against Microcystis aeruginosa surface antigen

<400> 4
cagatgtggt attcttgggt taagcaaaga ccggggcaag ggctggagtg gattggccag 60

cagtattggt ctacgccgcc gtggacg 87

<210> 5
<211> 29
<212> PRT
<213> Artificial Sequence

<220>
<223> VHCDR1 N-C sequence in reversed order of amino acid sequence of antibody mimetics against Microcystis aeruginosa surface antigen

<400> 5

```
Gln Met Trp Tyr Ser Trp Val Lys Gln Arg Pro Gly Gln Gly Leu Glu
1               5                   10                  15

Trp Ile Gly Gln Gln Tyr Trp Ser Thr Pro Pro Trp Thr
            20                  25
```

<210> 6
<211> 1965
<212> DNA
<213> Artificial Sequence

<220>
<223> Nucleotide sequence encoding anti-cyanobacteria polypeptide1

<400> 6

```
atgtctgacc ctgtacgtat tacaaatccc ggtgcagaat cgctggggta tgattcagat      60
ggccatgaaa ttatggccgt tgatatttat gtaaaccctc cacgtgtcga tgtctttcat     120
ggtaccccgc ctgcatggag ttccttcggg aacaaaacca tctggggcgg aaacgagtgg     180
gttgatgatt ccccaacccg aagtgatatc gaaaaaaggg acaaggaaat cacagcgtac     240
aaaaacacgc tcagcgcgca gcagaaagag aatgagaata agcgtactga agccggaaaa     300
cgcctctctg cggcgattgc tgcaagggaa aaagatgaaa acacactgaa aacactccgt     360
gccggaaacg cagatgccgc tgatattaca cgacaggagt tcagactcct gcaggcagag     420
ctgagagaat acggattccg tactgaaatc gccggatatg acgccctccg gctgcataca     480
gagagccgga tgctgtttgc tgatgctgat tctcttcgta tatctccccg ggaggccagg     540
tcgttaatcg aacaggctga aaaacggcag aaggatgcgc agaacgcaga caagaaggcc     600
gctgatatgc ttgctgaata cgagcgcaga aaaggtattc tggacacccg gttgtcagag     660
ctggaaaaaa atggcggggc agcccttgcc gttcttgatg cacaacaggc ccgtctgctc     720
gggcagcaga cacggaatga cagggccatt tcagaggccc ggaataaact cagttcagtg     780
acggaatcgc ttaacacggc ccgtaatgca ttaaccagag ctgaacaaca gctgacgcaa     840
cagaaaaaca cgcctgacgg caaaacgata gtttcccctg aaaaattccc ggggcgttca     900
tcaacaaatc attctattgt tgtgagcggt gatccgagat ttgccggtac gataaaaatc     960
acaaccagcg cagtcatcga taaccgtgca aacctgaatt atcttctgag ccattccggt    1020
ctggactata aacgcaatat tctgaatgac cggaatccgg tggtgacaga ggatgtggaa    1080
ggtgacaaga aaatttataa tgctgaagtt gctgaatggg ataagttacg gcaaagattg    1140
cttgatgcca gaaataaaat cacctctgct gaatctgcgg taaattcggc gagaaataac    1200
```

```
ctcagtgcca gaacaaatga gcaaaagcat gcaaatgacg ctcttaatgc cctgttgaag      1260
gaaaaagaga atatacgtaa ccagctttcc ggcatcaatc agaagatagc ggaagagaaa      1320
agaaaacagg atgaactgaa ggcaacgaaa gacgcaatta atttcacaac agagttcctg      1380
aaatcagttt cagaaaaata tggtgcaaaa gctgagcagt tagccagaga gatggccggg      1440
caggctaaag ggaagaaaat acgtaatgtt gaagaggcat taaaaacgta tgaaaagtac      1500
cgggctgaca ttaacaaaaa aattaatgca aaagatcgtg cagcgattgc cgcagccctt      1560
gagtctgtga agctgtctga tatatcgtct aatctgaaca gattcagtcg gggactggga      1620
tatgcaggaa aatttacaag tcttgctgac tggatcactg agtttggtaa ggctgtccgg      1680
acagagaact ggcgtcctct ttttgttaaa acagaaacca tcatagcagg caatgccgca      1740
acggctcttg tggcactggt cttcagtatt cttaccggaa gcgctttagg cattatcggg      1800
tatggtttac tgatggctgt caccggtgcg ctgattgatg aatcgcttgt ggaaaaagcg      1860
aataagttct ggggtatttc ttattggatg cagtgggtta agcaaagacc ggggcaaggg      1920
ctggagtgga ttggccagca gtattggtct acgccgccgt ggacg                      1965
```

<210> 7
<211> 654
<212> PRT
<213> Artificial Sequence

<220>
<223> Amino acid sequence of anti-cyanobacteria polypeptide 1

<400> 7

```
Ser Asp Pro Val Arg Ile Thr Asn Pro Gly Ala Glu Ser Leu Gly Tyr
1               5                   10                  15

Asp Ser Asp Gly His Glu Ile Met Ala Val Asp Ile Tyr Val Asn Pro
            20                  25                  30

Pro Arg Val Asp Val Phe His Gly Thr Pro Pro Ala Trp Ser Ser Phe
        35                  40                  45

Gly Asn Lys Thr Ile Trp Gly Gly Asn Glu Trp Val Asp Asp Ser Pro
    50                  55                  60

Thr Arg Ser Asp Ile Glu Lys Arg Asp Lys Glu Ile Thr Ala Tyr Lys
65                  70                  75                  80

Asn Thr Leu Ser Ala Gln Gln Lys Glu Asn Glu Asn Lys Arg Thr Glu
                85                  90                  95

Ala Gly Lys Arg Leu Ser Ala Ala Ile Ala Ala Arg Glu Lys Asp Glu
            100                 105                 110

Asn Thr Leu Lys Thr Leu Arg Ala Gly Asn Ala Asp Ala Ala Asp Ile
        115                 120                 125
```

Thr Arg Gln Glu Phe Arg Leu Leu Gln Ala Glu Leu Arg Glu Tyr Gly
130 135 140

Phe Arg Thr Glu Ile Ala Gly Tyr Asp Ala Leu Arg Leu His Thr Glu
145 150 155 160

Ser Arg Met Leu Phe Ala Asp Ala Asp Ser Leu Arg Ile Ser Pro Arg
165 170 175

Glu Ala Arg Ser Leu Ile Glu Gln Ala Glu Lys Arg Gln Lys Asp Ala
180 185 190

Gln Asn Ala Asp Lys Lys Ala Ala Asp Met Leu Ala Glu Tyr Glu Arg
195 200 205

Arg Lys Gly Ile Leu Asp Thr Arg Leu Ser Glu Leu Glu Lys Asn Gly
210 215 220

Gly Ala Ala Leu Ala Val Leu Asp Ala Gln Gln Ala Arg Leu Leu Gly
225 230 235 240

Gln Gln Thr Arg Asn Asp Arg Ala Ile Ser Glu Ala Arg Asn Lys Leu
245 250 255

Ser Ser Val Thr Glu Ser Leu Asn Thr Ala Arg Asn Ala Leu Thr Arg
260 265 270

Ala Glu Gln Gln Leu Thr Gln Gln Lys Asn Thr Pro Asp Gly Lys Thr
275 280 285

Ile Val Ser Pro Glu Lys Phe Pro Gly Arg Ser Ser Thr Asn His Ser
290 295 300

Ile Val Val Ser Gly Asp Pro Arg Phe Ala Gly Thr Ile Lys Ile Thr
305 310 315 320

Thr Ser Ala Val Ile Asp Asn Arg Ala Asn Leu Asn Tyr Leu Leu Ser
325 330 335

His Ser Gly Leu Asp Tyr Lys Arg Asn Ile Leu Asn Asp Arg Asn Pro
340 345 350

Val Val Thr Glu Asp Val Glu Gly Asp Lys Lys Ile Tyr Asn Ala Glu
355 360 365

Val Ala Glu Trp Asp Lys Leu Arg Gln Arg Leu Leu Asp Ala Arg Asn
370 375 380

Lys Ile Thr Ser Ala Glu Ser Ala Val Asn Ser Ala Arg Asn Asn Leu
385 390 395 400

Ser Ala Arg Thr Asn Glu Gln Lys His Ala Asn Asp Ala Leu Asn Ala
405 410 415

Leu Leu Lys Glu Lys Glu Asn Ile Arg Asn Gln Leu Ser Gly Ile Asn
420 425 430

Gln Lys Ile Ala Glu Glu Lys Arg Lys Gln Asp Glu Leu Lys Ala Thr
435 440 445

Lys Asp Ala Ile Asn Phe Thr Thr Glu Phe Leu Lys Ser Val Ser Glu
450 455 460

Lys Tyr Gly Ala Lys Ala Glu Gln Leu Ala Arg Glu Met Ala Gly Gln
465 470 475 480

Ala Lys Gly Lys Lys Ile Arg Asn Val Glu Glu Ala Leu Lys Thr Tyr
485 490 495

Glu Lys Tyr Arg Ala Asp Ile Asn Lys Lys Ile Asn Ala Lys Asp Arg
500 505 510

Ala Ala Ile Ala Ala Ala Leu Glu Ser Val Lys Leu Ser Asp Ile Ser
515 520 525

Ser Asn Leu Asn Arg Phe Ser Arg Gly Leu Gly Tyr Ala Gly Lys Phe
530 535 540

Thr Ser Leu Ala Asp Trp Ile Thr Glu Phe Gly Lys Ala Val Arg Thr
545 550 555 560

Glu Asn Trp Arg Pro Leu Phe Val Lys Thr Glu Thr Ile Ile Ala Gly
565 570 575

Asn Ala Ala Thr Ala Leu Val Ala Leu Val Phe Ser Ile Leu Thr Gly
580 585 590

Ser Ala Leu Gly Ile Ile Gly Tyr Gly Leu Leu Met Ala Val Thr Gly
595 600 605

Ala Leu Ile Asp Glu Ser Leu Val Glu Lys Ala Asn Lys Phe Trp Gly
610 615 620

Ile Ser Tyr Trp Met Gln Trp Val Lys Gln Arg Pro Gly Gln Gly Leu
625 630 635 640

Glu Trp Ile Gly Gln Gln Tyr Trp Ser Thr Pro Pro Trp Thr
645 650

<210> 8
<211> 1965
<212> DNA

<213>  Artificial Sequence

<220>
<223>  Nucleotide sequence encoding antifugal polypeptide 2

<400>  8
atgtctgacc ctgtacgtat tacaaatccc ggtgcagaat cgctggggta tgattcagat    60
ggccatgaaa ttatggccgt tgatatttat gtaaaccctc cacgtgtcga tgtctttcat   120
ggtaccccgc ctgcatggag ttccttcggg aacaaaacca tctggggcgg aaacgagtgg   180
gttgatgatt ccccaacccg aagtgatatc gaaaaaaggg acaaggaaat cacagcgtac   240
aaaaacacgc tcagcgcgca gcagaaagag aatgagaata agcgtactga agccggaaaa   300
cgcctctctg cggcgattgc tgcaagggaa aaagatgaaa acacactgaa aacactccgt   360
gccggaaacg cagatgccgc tgatattaca cgacaggagt tcagactcct gcaggcagag   420
ctgagagaat acggattccg tactgaaatc gccggatatg acgccctccg gctgcataca   480
gagagccgga tgctgtttgc tgatgctgat tctcttcgta tatctccccg ggaggccagg   540
tcgttaatcg aacaggctga aaaacggcag aaggatgcgc agaacgcaga caagaaggcc   600
gctgatatgc ttgctgaata cgagcgcaga aaaggtattc tggacacccg gttgtcagag   660
ctggaaaaaa atggcggggc agcccttgcc gttcttgatg cacaacaggc ccgtctgctc   720
gggcagcaga cacggaatga cagggccatt tcagaggccc ggaataaact cagttcagtg   780
acggaatcgc ttaacacggc ccgtaatgca ttaaccagag ctgaacaaca gctgacgcaa   840
cagaaaaaca cgcctgacgg caaaacgata gtttcccctg aaaaattccc ggggcgttca   900
tcaacaaatc attctattgt tgtgagcggt gatccgagat ttgccggtac gataaaaatc   960
acaaccagcg cagtcatcga taaccgtgca aacctgaatt atcttctgag ccattccggt  1020
ctggactata aacgcaatat tctgaatgac cggaatccgg tggtgacaga ggatgtggaa  1080
ggtgacaaga aaatttataa tgctgaagtt gctgaatggg ataagttacg gcaaagattg  1140
cttgatgcca gaaataaaat cacctctgct gaatctgcgg taaattcggc gagaaataac  1200
ctcagtgcca gaacaaatga gcaaaagcat gcaaatgacg ctcttaatgc cctgttgaag  1260
gaaaaagaga atatacgtaa ccagctttcc ggcatcaatc agaagatagc ggaagagaaa  1320
agaaaacagg atgaactgaa ggcaacgaaa gacgcaatta atttcacaac agagttcctg  1380
aaatcagttt cagaaaaata tggtgcaaaa gctgagcagt tagccagaga gatggccggg  1440
caggctaaag ggaagaaaat acgtaatgtt gaagaggcat taaaaacgta tgaaaagtac  1500
cgggctgaca ttaacaaaaa aattaatgca aaagatcgtg cagcgattgc cgcagccctt  1560
gagtctgtga agctgtctga tatatcgtct aatctgaaca gattcagtcg gggactggga  1620
tatgcaggaa aatttacaag tcttgctgac tggatcactg agtttggtaa ggctgtccgg  1680
acagagaact ggcgtcctct tttgttaaa acagaaacca tcatagcagg caatgccgca   1740
acggctcttg tggcactggt cttcagtatt cttaccggaa gcgctttagg cattatcggg  1800
tatggtttac tgatggctgt caccggtgcg ctgattgatg aatcgcttgt ggaaaaagcg  1860

```
aataagttct ggggtattca gatgtggtat tcttgggtta agcaaagacc ggggcaaggg    1920
ctggagtgga ttggccagca gtattggtct acgccgccgt ggacg                    1965

<210>  9
<211>  654
<212>  PRT
<213>  Artificial Sequence

<220>
<223>  Amino acid sequence of anti-cyanobacteria polypeptide 2

<400>  9
Ser Asp Pro Val Arg Ile Thr Asn Pro Gly Ala Glu Ser Leu Gly Tyr
1               5                   10                  15

Asp Ser Asp Gly His Glu Ile Met Ala Val Asp Ile Tyr Val Asn Pro
            20                  25                  30

Pro Arg Val Asp Val Phe His Gly Thr Pro Pro Ala Trp Ser Ser Phe
        35                  40                  45

Gly Asn Lys Thr Ile Trp Gly Gly Asn Glu Trp Val Asp Asp Ser Pro
    50                  55                  60

Thr Arg Ser Asp Ile Glu Lys Arg Asp Lys Glu Ile Thr Ala Tyr Lys
65                  70                  75                  80

Asn Thr Leu Ser Ala Gln Gln Lys Glu Asn Glu Asn Lys Arg Thr Glu
                85                  90                  95

Ala Gly Lys Arg Leu Ser Ala Ala Ile Ala Ala Arg Glu Lys Asp Glu
            100                 105                 110

Asn Thr Leu Lys Thr Leu Arg Ala Gly Asn Ala Asp Ala Ala Asp Ile
        115                 120                 125

Thr Arg Gln Glu Phe Arg Leu Leu Gln Ala Glu Leu Arg Glu Tyr Gly
    130                 135                 140

Phe Arg Thr Glu Ile Ala Gly Tyr Asp Ala Leu Arg Leu His Thr Glu
145                 150                 155                 160

Ser Arg Met Leu Phe Ala Asp Ala Asp Ser Leu Arg Ile Ser Pro Arg
                165                 170                 175

Glu Ala Arg Ser Leu Ile Glu Gln Ala Glu Lys Arg Gln Lys Asp Ala
            180                 185                 190

Gln Asn Ala Asp Lys Lys Ala Ala Asp Met Leu Ala Glu Tyr Glu Arg
        195                 200                 205

Arg Lys Gly Ile Leu Asp Thr Arg Leu Ser Glu Leu Glu Lys Asn Gly
    210                 215                 220
```

Gly Ala Ala Leu Ala Val Leu Asp Ala Gln Gln Ala Arg Leu Leu Gly
225 230 235 240

Gln Gln Thr Arg Asn Asp Arg Ala Ile Ser Glu Ala Arg Asn Lys Leu
245 250 255

Ser Ser Val Thr Glu Ser Leu Asn Thr Ala Arg Asn Ala Leu Thr Arg
260 265 270

Ala Glu Gln Gln Leu Thr Gln Gln Lys Asn Thr Pro Asp Gly Lys Thr
275 280 285

Ile Val Ser Pro Glu Lys Phe Pro Gly Arg Ser Ser Thr Asn His Ser
290 295 300

Ile Val Val Ser Gly Asp Pro Arg Phe Ala Gly Thr Ile Lys Ile Thr
305 310 315 320

Thr Ser Ala Val Ile Asp Asn Arg Ala Asn Leu Asn Tyr Leu Leu Ser
325 330 335

His Ser Gly Leu Asp Tyr Lys Arg Asn Ile Leu Asn Asp Arg Asn Pro
340 345 350

Val Val Thr Glu Asp Val Glu Gly Asp Lys Lys Ile Tyr Asn Ala Glu
355 360 365

Val Ala Glu Trp Asp Lys Leu Arg Gln Arg Leu Leu Asp Ala Arg Asn
370 375 380

Lys Ile Thr Ser Ala Glu Ser Ala Val Asn Ser Ala Arg Asn Asn Leu
385 390 395 400

Ser Ala Arg Thr Asn Glu Gln Lys His Ala Asn Asp Ala Leu Asn Ala
405 410 415

Leu Leu Lys Glu Lys Glu Asn Ile Arg Asn Gln Leu Ser Gly Ile Asn
420 425 430

Gln Lys Ile Ala Glu Glu Lys Arg Lys Gln Asp Glu Leu Lys Ala Thr
435 440 445

Lys Asp Ala Ile Asn Phe Thr Thr Glu Phe Leu Lys Ser Val Ser Glu
450 455 460

Lys Tyr Gly Ala Lys Ala Glu Gln Leu Ala Arg Glu Met Ala Gly Gln
465 470 475 480

Ala Lys Gly Lys Lys Ile Arg Asn Val Glu Glu Ala Leu Lys Thr Tyr
485 490 495

Glu Lys Tyr Arg Ala Asp Ile Asn Lys Lys Ile Asn Ala Lys Asp Arg
500 505 510

Ala Ala Ile Ala Ala Ala Leu Glu Ser Val Lys Leu Ser Asp Ile Ser
515 520 525

Ser Asn Leu Asn Arg Phe Ser Arg Gly Leu Gly Tyr Ala Gly Lys Phe
530 535 540

Thr Ser Leu Ala Asp Trp Ile Thr Glu Phe Gly Lys Ala Val Arg Thr
545 550 555 560

Glu Asn Trp Arg Pro Leu Phe Val Lys Thr Glu Thr Ile Ile Ala Gly
565 570 575

Asn Ala Ala Thr Ala Leu Val Ala Leu Val Phe Ser Ile Leu Thr Gly
580 585 590

Ser Ala Leu Gly Ile Ile Gly Tyr Gly Leu Leu Met Ala Val Thr Gly
595 600 605

Ala Leu Ile Asp Glu Ser Leu Val Glu Lys Ala Asn Lys Phe Trp Gly
610 615 620

Ile Gln Met Trp Tyr Ser Trp Val Lys Gln Arg Pro Gly Gln Gly Leu
625 630 635 640

Glu Trp Ile Gly Gln Gln Tyr Trp Ser Thr Pro Pro Trp Thr
645 650

<210> 10
<211> 69
<212> DNA
<213> Artificial Sequence

<220>
<223> 1. 5¡¯-3¡¯Sequences of double strand oligonucleotides designed for the preparation on genes of antibody mimetics of the plasmid pCHCcyanoMA1

<400> 10
gcgaataagt tctggggtat ttcttattgg atgcagtggg ttaagcaata aataaaatat 60

aagacaggc 69

<210> 11
<211> 69
<212> DNA
<213> Artificial Sequence

<220>
<223> 1. 3¡¯-5¡¯Sequences of double strand oligonucleotides designed for the preparation on genes of antibody mimetics of the plasmid pCHCcyanoMA1

<400> 11
gcctgtctta tattttattt attgcttaac ccactgcatc caataagaaa taccccagaa 60

cttattcgc 69

<210> 12
<211> 75
<212> DNA
<213> Artificial Sequence

<220>
<223> 2. 5'-3'Sequences of double strand oligonucleotides designed for the preparation on genes of antibody mimetics of the plasmid pCHCcyanoMA1

<400> 12
tattggatgc agtgggttaa gcaaagaccg gggcaagggc tggagtggat tggctaaata 60

aaatataaga caggc 75

<210> 13
<211> 75
<212> DNA
<213> Artificial Sequence

<220>
<223> 1. 3¡¯-5¡¯Sequences of double strand oligonucleotides designed for the preparation on genes of antibody mimetics of the plasmid pCHCcyanoMA1

<400> 13
gcctgtctta tattttattt agccaatcca ctccagccct tgccccggtc tttgcttaac 60

ccactgcatc caata 75

<210> 14
<211> 75
<212> DNA
<213> Artificial Sequence

<220>
<223> 3. 5¡¯-3¡¯Sequences of double strand oligonucleotides designed for the preparation on genes of antibody mimetics of the plasmid pCHCcyanoMA1

<400> 14
gggcaagggc tggagtggat tggccagcag tatyggtcta cgcccccgtg gacgtaaata 60

aaatataaga caggc 75

<210> 15
<211> 75
<212> DNA
<213> Artificial Sequence

<220>
<223> 3. 3¡¯-5¡¯Sequences of double strand oligonucleotides designed for the preparation on genes of antibody mimetics of the plasmid pCHCcyanoMA1

<400> 15
gcctgtctta tattttattt acgtccacgg gggcgtagac caatactgct ggccaatcca 60

ctccagccct tgccc 75